(19) Europäisches Patentamt
European Patent Office
Office européen des brevets

(11) EP 2 356 118 B1

(12) **EUROPÄISCHE PATENTSCHRIFT**

(45) Veröffentlichungstag und Bekanntmachung des Hinweises auf die Patenterteilung:
**17.07.2013 Patentblatt 2013/29**

(21) Anmeldenummer: **09793454.1**

(22) Anmeldetag: **23.11.2009**

(51) Int Cl.:
*C07D 471/06* (2006.01) *C09K 11/06* (2006.01)
*H01L 51/00* (2006.01) *H01L 51/46* (2006.01)

(86) Internationale Anmeldenummer:
**PCT/EP2009/008330**

(87) Internationale Veröffentlichungsnummer:
**WO 2010/057669 (27.05.2010 Gazette 2010/21)**

(54) **SYSTEME ZUR LICHTGETRIEBENEN TRENNUNG VON LADUNGEN**
SYSTEMS FOR THE LIGHT-INDUCED SEPARATION OF CHARGES
SYSTÈMES DE SÉPARATION DE CHARGES PRODUITE PAR LA LUMIÈRE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **21.11.2008 DE 102008058454**
**09.10.2009 DE 102009048906**

(43) Veröffentlichungstag der Anmeldung:
**17.08.2011 Patentblatt 2011/33**

(73) Patentinhaber: **cynora GmbH**
**76344 Eggenstein-Leopoldshafen (DE)**

(72) Erfinder:
- **LANGHALS, Heinz**
**85521 Ottobrunn (DE)**
- **OBERMEIER, Andreas**
**85232 Bergkirchen (DE)**

(74) Vertreter: **Hoppe, Georg Johannes**
**darani Anwaltskanzlei**
**Beuckestrasse 20**
**14163 Berlin (DE)**

(56) Entgegenhaltungen:

- **JANSSEN R A J ET AL: "Alternating Oligo(p-phenylene vinylene)-Perylene Bisimide Copolymers: Synthesis, Photophysics, and Photovoltaic Properties of a New Class of Donor-Acceptor Materials" JOURNAL OF THE AMERICAN CHEMICAL SOCIETY, Bd. 125, 18. Juni 2003 (2003-06-18), Seiten 8625-8638, XP002576479**
- **WASIELEWSKI M R ET AL: "Ultrafast Aggregate-to-Aggregate Energy Transfer within Self-assembled Light-Harvesting Columns of Zinc Phthalocyanine Tetrakis(perylenediimide)" JOURNAL OF THE AMERICAN CHEMICAL SOCIETY, Bd. 126, 14. August 2004 (2004-08-14), Seiten 10810-10811, XP002576480**
- **FLAMIGNI L, GRYKO D T, LANGHALS H ET AL: "New and Efficient Arrays for Photoinduced Charge Separation Based on Perylene Bisimide and Corroles" CHEMISTRY - A EUROPEAN JOURNAL, Bd. 14, 9. Oktober 2007 (2007-10-09), Seiten 169-183, XP002576481**
- **LANGHALS H, OBERMEIER A: "A Click Reaction for Fluorescent Labelling: Application of the 1,3-Dipolar Cycloaddition Reaction" EUROPEAN JOURNAL OF ORGANIC CHEMISTRY, 7. November 2008 (2008-11-07), Seiten 6144-6151, XP002576482**
- **LANGHALS H, JONA W: "THE IDENTIFICATION OF CARBONYL COMPOUNDS BY FLUORESCENCE: A NOVEL CARBONYL-DERIVATIZING REAGENT" CHEMISTRY - A EUROPEAN JOURNAL, WILEY - V C H VERLAG GMBH & CO. KGAA, WEINHEIM, DE LNKD- DOI: 10.1002/(SICI)1521-3765(19981102)4:11< 2110:: AID-CHEM2110>3.3.CO;2-A, Bd. 4, Nr. 11, 1. Januar 1998 (1998-01-01) , Seiten 2110-2116, XP000973670 ISSN: 0947-6539**
- **LANGHALS H, BLANKE P: "An approach to novel NIR dyes utilising alpha-effect donor groups" DYES AND PIGMENTS, ELSEVIER APPLIED SCIENCE PUBLISHERS. BARKING, GB LNKD- DOI:10.1016/S0143-7208(03)00083-4, Bd. 59, Nr. 2, 1. November 2003 (2003-11-01), Seiten 109-116, XP004452626 ISSN: 0143-7208**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 356 118 B1

Bemerkungen:

Die Akte enthält technische Angaben, die nach dem Eingang der Anmeldung eingereicht wurden und die nicht in dieser Patentschrift enthalten sind.

## Beschreibung

Die lichtgetriebene Trennung von elektrischen Ladungen erlangt in der wissenschaftlichen Grundlagenforschung und in der Technik ein wachsendes Interesse. Hier besteht die Aussicht, Solarenergie effizient zu sammeln und in elektrische Energie umzuwandeln. Als lichtabsorbierende Strukturen sind organische Materialien für künftige Entwicklungen ausgesprochen interessant, weil mit ihnen farbstarke Chromophore realisiert werden können, die auf unkomplizierte Weise in weiten Grenzen abgewandelt und auf die jeweiligen Erfordernisse angepasst werden können. Darüber hinaus lassen sich organische Materialien problemlos recyclen oder entsorgen, ein Gesichtspunkt, der für längerfristige Entwicklungen immer bedeutsamer werden wird. Bisher steht allerdings nur eine begrenzte Zahl solcher Verbindungen zur Verfügung. Die Langzeitstabilität derartiger Systeme ist ein ungelöstes Problem. Ein universelles, lichtechtes System zur lichtinduzierten Erzeugung von Ladungstrennung brächte einen erheblichen Fortschritt.

Für lichtgetriebene Ladungstrennungen benötigt man eine lichtabsorbierende Einheit - im folgenden auch Chromophor genannt - und eine Struktur, die die Ladungstrennung bewirkt. Grundsätzlich können beide Einheiten aus Chromophoren aufgebaut sein, wie dies in vorangegangenen Arbeiten realisiert wurde (L. Flamigni, B. Ventura, M. Tasior, T. Becherer, H. Langhals, D. T. Gryko, Chem. Eur. J. 2008, 14, 169-183; M. Tasior, D. T. Gryko, Jing Shen, K. M. Kadish, T. Becherer, H. Langhals, B. Ventura, L. Flamigni, J. Phys. Chem. 2008, 112, 19699-19709; JP 8065635 A). Hier besteht jedoch das grundsätzliche Problem, dass beide Chromophore sowohl auf die Lichtabsorption als auch die Ladungstrennung optimiert sein müssen. Eine solche Strategie führt vielfach zu Kompromissen, die eingegangen werden müssen. Zudem besteht das grundlegende Problem, dass die Extinktionskoeffizienten der Einzelchromophore aufeinander abgestimmt sein müssen, weil die Chromophore miteinander verknüpft sind, so dass das Lichtabsorptionsvermögen der endgültigen Anordnung nicht durch die relativen Konzentration der Komponenten gesteuert werden kann.

Auch Strukturen, die aus einem Chromophor und einem farblosen Molekülteil, der die Ladungstrennung bewirken soll, als Substituenten am Chromophor bestehen, müssen noch weiter entwickelt werden. Für eine Elektronenübertragung nach der optischen Anregung wird ein hoch liegendes Orbital benötigt, gleichgültig ob $\pi$- oder n-Orbital, so z.B. wie das n-Orbital von freien Aminen. Durch die optischen Anregung entsteht eine Elektronlücke im HOMO des Farbstoffs, die dann durch die Elektronenübertragung vom hochliegenden Orbital des Substituenten ausgehend gefüllt wird, so dass eine Rückkehr des angeregten Elektrons, z.B. unter Fluoreszenzlicht-Abgabe, unmöglich wird. Hierdurch ist die Fluoreszenzquenchung gleichermaßen eine Konsequenz und ein sehr guter Indikator für die Elektronenübertragungsreaktion. Eine Aminogruppe direkt an das Carbonsäureimid-Stickstoffatom gebunden führt zu einer Fluoreszenzquenchung (H. Langhals, W. Jona, Chem. Eur.J. 1998, 4, 2110-2116) und belegt diesen Prozess. Allerdings sind die Aminogruppe und der Chromophor sehr nah benachbart, so dass die Rückübertragung schnell erfolgt und damit kaum eine Nutzung der Ladungstrennung möglich ist. Bringt man einen Spacer zwischen die Aminogruppe und den Chromophor, dann kommt enttäuschenderweise dieser Elektronenübertragungsprozess vollständig zum Erliegen und die Substanzen fluoreszieren mit nahezu 100% (H. Langhals, A. Obermeier, Eur. J. Org. Chem. 2008, 36, 6144-6151). Hier stellt sich die Frage, ob man die Elektronendonor-Eigenschaften der Gruppe so weit erhöhen kann, dass die Elektronenübertragung auch bei größerem Abstand wieder eingeschaltet wird.

Die Aufgabe der vorliegenden Erfindung war es vor diesem Hintergrund, organische Strukturen zu entwickeln, bei denen es auf effiziente Weise gelingt eine lichtgetriebene Ladungstrennung zu erzielen. Hierbei war die Lichtechtheit solcher Strukturen von zentraler Bedeutung.

Gelöst wurde diese Aufgabe erfindungsgemäß mit Verbindungen der folgenden allgemeinen Formel I, die auch als Perylenbisimid-Dyaden bezeichnet werden.

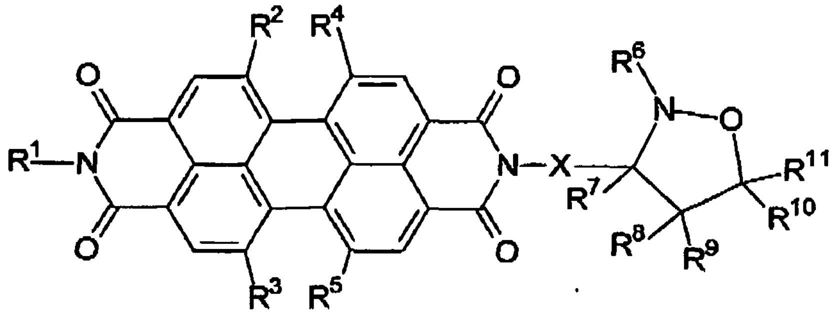

(I)

Die Bedeutung der Reste $R^1$ bis $R^{11}$ sowie der Gruppierung X ist nachstehend näher erläutert.

Darüber hinaus stellt die vorliegend Erfindung Perylentetracarbonsäurebisimid-Nitrone der allgemeinen Formel

(II) bereit,

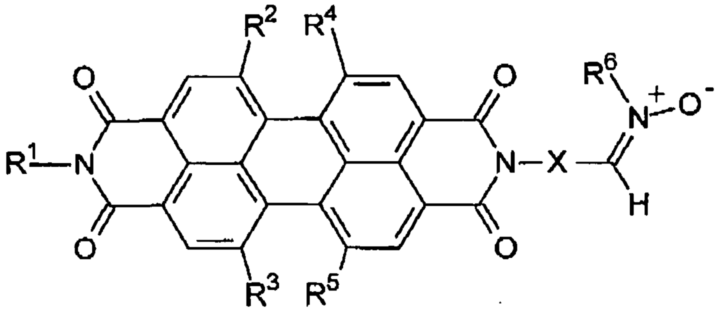

(II)

in der die Reste $R^1$ bis $R^6$ und X unabhängig voneinander die gleiche Bedeutung haben wie für Verbindungen der Formel (I).

α-Effekt-Verbindungen der allgemeinen Formel (III) können als Elektronendonorgruppen in lichtgetriebenen Systemen zur Ladungstrennung verwendet werden,

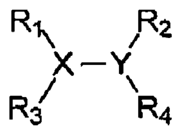

(III)

wobei X und Y sowie die Reste $R_1$ bis $R_4$ ebenfalls nachstehend erläutert werden.

Schließlich betrifft die Erfindung nach einem weiteren Aspekt die Verwendung von Isoxazolidinen der allgemeinen Formel (IV) als Elektronendonorgruppen in lichtgetriebenen Systemen zur Ladungstrennung,

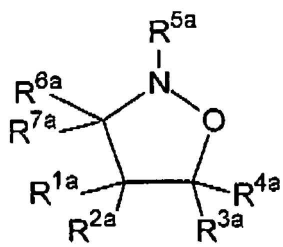

(IV)

wobei die Reste $R^{1a}$ bis $R^{7a}$ ebenfalls nachstehend erläutert werden.

Figur 1 zeigt beispielhaft die Synthese der Nitrone **3** und **4** und Reaktion von **4** mit Styrol zu den Oxazolidinen **5a** und **5b.**

Figur 2 zeigt beispielhaft die Synthese der Oxazolidine **6** bis **9.**

Figur 3 zeigt beispielhaft die Synthese des Nitrons **11** und der Oxazolidine **12a** und **12b.**

Figur 4 zeigt Grundzustände und elektronisch angeregte Zustände von **5** und **12.**

Figur 5 zeigt UV/Vis Absorptions- und Fluoreszenzspektren der Osoxazolidine (oberhalb von 400 nm deckungsgleich) verglichen mit **1a** (R = 1-Hexylheptyl), das um ca. ein halbes Nanometer kürzerwellig erscheint. Bei 300 nm

von oben nach unten: **12**, **1a**, **5**, **6** und **7.**

Es zeigte sich im Rahmen der Erfindung, dass es vorteilhaft ist einen einzelnen Chromophor mit einer farblosen Struktur zu verknüpfen, um die Ladungstrennung zu erreichen.

Als besonders geeignete Chromophore wurden die Perylen-3,4:9,10-tetracarbonsäurebisimide identifiziert, die schematisch in der folgenden Formel **1** dargestellt sind,

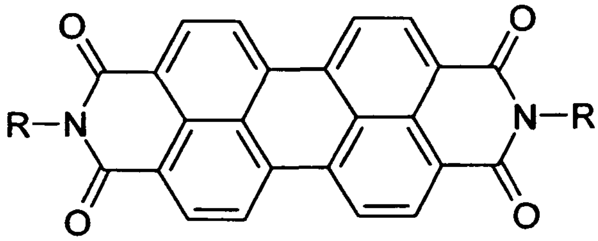

**1**

sowie Derivate davon, wie nachstehend ausgeführt. Sie zeichnen sich durch hohe Absorptionskoeffizienten, ihre große Lichtechtheit und chemische Beständigkeit aus (H. Langhals, Helv. Chim. Acta. 2005, 88, 1309-1343; H. Langhals, Heterocycles 1995, 40, 477-500; H. Langhals, Molecular Devices. Chiral, Bichromophoric Silicones: Ordering Principles in Complex Molecules in F. Ganachaud, S. Boileau, B. Boury (eds.), Silicon Based Polymers, p. 51-63, Springer, 2008, ISBN 978-1-4020-8527-7, e-ISBN 978-1-4020-8528-4).

Ihre hohen Fluoreszenzquantenausbeuten (H. Langhals, J. Karolin, L. B.-Ä. Johansson, J. Chem. Soc., Faraday Trans. 1998, 94, 2919-2922) von nahezu 100% sind von Vorteil für die Entwicklung von Systemen mit lichtgetriebener Ladungstrennung, denn dadurch bleibt die optische Anregungsenergie für die in der Folge ablaufenden Prozesse erhalten. Die Stickstoffatome von **1** sind ideale Verknüpfungsstellen für den Aufbau der weiteren Struktur. Um die Löslichkeit der Perylenbisimide zu fördern, kann z.B. für einen der Reste R ein löslichkeitssteigernder 1-Hexylheptyl-Rest ("Schwalbenschwanz-Rest"; Die Verbindung 1a hat die vorstehend dargestellte Formel 1, wobei beide Reste R für einen 1-Hexylheptyl-Rest stehen) eingesetzt werden (S. Demmig, H. Langhals, Chem. Ber. 1988, 121, 225-230; H. Langhals, S. Demmig, T. Potrawa, J. Prakt. Chem. 1991, 333, 733-748). Die Verknüpfung zu den weiteren funktionalen Strukturen kann beispielsweise mit dem zweiten Rest R vorgenommen werden. Zusätzlich können weitere Substituenten am Perylengerüst eingeführt werden.

In den eingesetzten farblosen Gruppen zum Bewirken einer Ladungstrennung, die in erfindungsgemäßen Systemen zur lichtgetriebenen Ladungstrennung mit einem Chromphor verbunden sind, gelang es im Rahmen der Erfindung, den $\alpha$-Effekt zu nutzen, um eine Fluoreszenzquenchung der Ladungstrennung zu verhindern. Hierzu wird zum Bewirken der Ladungstrennung eine Gruppe eingesetzt mit zwei direkt aneinander gebundenen Atomen, die beide freie Elektronenpaare tragen. Hier ist insbesondere eine Kombination von Stickstoff mit Sauerstoff von Interesse. Durch den Einsatz von Isoxazolidinen (P. Grünanger, P. Vita-Finzi, Isoxazolidines, in The Chemistry of Heterocyclic compounds (E. C. Tayloer, A. Weissberger, Editoren) Vol. 49/1, S. 649-877, Wiley, New York 1991, ISBN 0-471-02233-0; S. Cicchi, F. M. Cordero, D. Giomi, Five-membered ring systems: with O & N atoms in Progress in Heterocyclic Chemistry 2003, 15, 261-283; Y. Takeuchi, F. Furusaki, The chemistry of isoxazolidines in Advances in Heterocyclic Chemistry 1977, 21, 207-251) kann einer derartigen Struktur durch den Einbau in einen Ring zusätzliche Stabilität verliehen werden.

Wie oben ausgeführt stellt die vorliegende Erfindung nach einem ersten Aspekt Perylenbisimid-Dyaden der allgemeinen Formel (I) bereit.

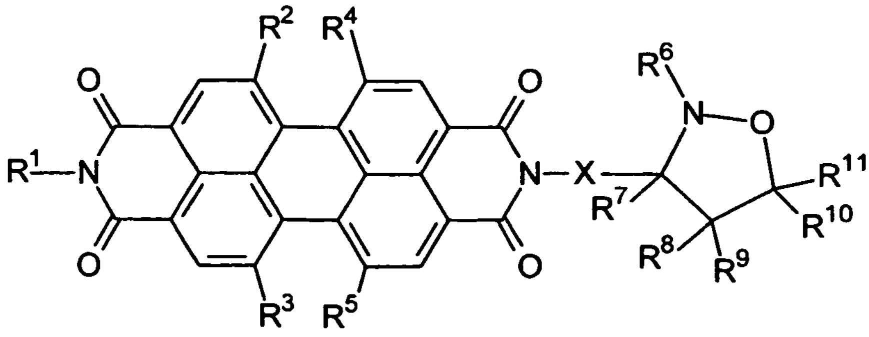

(I)

Die Reste $R^1$ bis $R^{11}$ sind in dieser Formel gleich oder verschieden und stehen unabhängig voneinander für Wasserstoff oder einen linearen Alkylrest mit mindestens einem und höchstens 37C-Atomen. In dem Alkylrest können eine bis 10 $CH_2$-Einheiten unabhängig voneinander ersetzt sein durch eine Carbonylgruppe, ein Sauerstoffatom, Schwefelatom, Selenatom, Telluratom, eine *cis*- oder *trans*-CH=CH-Gruppe, bei der eine CH-Einheit auch durch ein Stickstoffatom ersetzt sein kann, eine acetylenische C≡C-Gruppe, einen divalenten Phenylrest (z.B. 1,2-, 1,3- oder 1,4-Phenylrest), divalenten Pyridinrest (z.B. 2,3-, 2,4-, 2,5-, 2,6-, 3,4- oder 3,5- Pyridinrest), divalenten Thiophenrest (z.B. 2,3-, 2,4-, 2,5- oder 3,4- Thiophenrest), divalenten Naphthalinrest (z.B. 1,2-, 1,3-, 1,4-, 1,5-, 1,6-, 1,7-, 1,8-, 2,3-, 2,6- oder 2,7-Naphthalinrest), bei dem eine oder zwei CH-Gruppen durch Stickstoffatome ersetzt sein können, und einen divalenten Anthracenrest (z.B. 1,2-, 1,3-, 1,4-, 1,5-, 1,6-, 1,7-, 1,8-, 1,9-, 1,10-, 2,3-, 2,6-, 2,7-, 2,9-, 2,10- oder 9,10-Anthracenrest), bei dem eine oder zwei CH-Gruppen durch Stickstoffatome ersetzt sein können. Bis zu 12 einzelne Wasserstoffatome der $CH_2$-Gruppen können jeweils unabhängig voneinander auch an gleichen C-Atomen ersetzt sein durch die Halogene Fluor, Chlor, Brom oder Iod, eine Cyanogruppe oder eine lineare Alkylkette mit bis zu 18 C-Atomen, bei der eine bis 6 $CH_2$-Einheiten unabhängig voneinander ersetzt sein können durch eine Carbonylgruppe, ein Sauerstoffatom, Schwefelatom, Selenatom, Telluratom, eine *cis*- oder *trans*-CH=CH-Gruppe, bei der eine CH-Einheit durch ein Stickstoffatom ersetzt sein kann, eine acetylenische C≡C-Gruppe, einen divalenten Phenylrest (z.B. 1,2-, 1,3- oder 1,4-Phenylrest), einen divalenten Pyridinrest (z.B. 2,3-, 2,4-, 2,5-, 2,6-, 3,4- oder 3,5- Pyridinrest), einen divalenten Thiophenrest (z.B. 2,3-, 2,4-, 2,5- oder 3,4- Thiophenrest), einen divalenten Naphthalinrest (z.B. 1,2-, 1,3-, 1,4-, 1,5-, 1,6-, 1,7-, 1,8-, 2,3-, 2,6- oder 2,7- Naphthalinrest), bei dem eine oder zwei CH-Gruppen durch Stickstoffatome ersetzt sein können, und einen divalenten Anthracenrest (z.B. 1,2-, 1,3-, 1,4-, 1,5-, 1,6-, 1,7-, 1,8-, 1,9-, 1,10-, 2,3-, 2,6-, 2,7-, 2,9-, 2,10- oder 9,10- Anthracenrest), bei denen eine oder zwei CH-Gruppen durch Stickstoffatome ersetzt sein können. Bis zu 12 einzelne Wasserstoffatome der $CH_2$-Gruppen in einem Alkylrest können jeweils unabhängig voneinander auch an gleichen C-Atomen ersetzt sein durch die Halogene Fluor, Chlor, Brom oder Iod, eine Cyanogruppe oder eine lineare Alkylkette mit bis zu 18 C-Atomen, bei der eine bis 6 $CH_2$-Einheiten unabhängig voneinander ersetzt sein können durch eine Carbonylgruppe, ein Sauerstoffatom, Schwefelatom, Selenatom, Telluratom, eine *cis*- oder *trans*-CH=CH-Gruppe, bei der eine CH-Einheit durch ein Stickstoffatom ersetzt sein kann, eine acetylenische C≡C-Gruppe, einen divalenten Phenylrest (z.B. 1,2-, 1,3- oder 1,4-Phenylrest), einen divalenten Pyridinrest (z.B. 2,3-, 2,4-, 2,5-, 2,6-, 3,4- oder 3,5-Pyridinrest), einen divalente Thiophenrest (z.B. 2,3-, 2,4-, 2,5- oder 3,4- Thiophenrest), einen divalenten Naphthalinrest (z.B. 1,2-, 1,3-, 1,4-, 1,5-, 1,6-, 1,7-, 1,8-, 2,3-, 2,6- oder 2,7- Naphthalinrest), bei dem ein oder zwei CH-Gruppen durch Stickstoffatome ersetzt sein können, und einen divalenten Anthracenrest (z.B. 1,2-, 1,3-, 1,4-, 1,5-, 1,6-, 1,7-, 1,8-, 1,9-, 1,10-, 2,3-, 2,6-, 2,7-, 2,9-, 2,10- oder 9,10- Anthracenrest), bei dem eine oder zwei CH-Gruppen durch Stickstoffatome ersetzt sein können. $CH_2$-Gruppen, an denen wie vorstehend beschrieben ein Wasserstoffatom ersetzt ist, können auch unter Bildung eines Rings miteinander verknüpft sein, d.h. statt Substituenten zu tragen, können die freien Valenzen der Methingruppen bzw. der quartären C-Atome paarweise verknüpft werden, so dass Ringe entstehen, wie z.B. Cyclohexanringe. Die Reste $R^1$ bis $R^5$ und $R^7$ bis $R^{11}$ können außerdem unabhängig voneinander die Halogenatome F, Cl, Br oder I oder CN bedeuten.

X bedeutet in Formel (I) eine bis 12 $CH_2$-Einheiten, bei denen unabhängig voneinander eine oder mehrere ersetzt sein können durch jeweils eine Carbonylgruppe, ein Sauerstoffatom, Schwefelatom, Selenatom, Telluratom, eine *cis*- oder *trans*-CH=CH-Gruppe, bei der eine CH-Einheit auch durch ein Stickstoffatom ersetzt sein kann, eine acetylenische C≡C-Gruppe, einen divalenten Phenylrest (z.B. 1,2-, 1,3- oder 1,4- Phenylrest), einen divalenten Pyridinrest (z.B. 2,3-, 2,4-, 2,5-, 2,6-, 3,4- oder 3,5- Pyridinrest), einen divalenten Thiophenrest (z.B. 2,3-, 2,4-, 2,5- oder 3,4-Thiophenrest), einen divalenten Naphthalinrest (z.B. 1,2-, 1,3-, 1,4-, 1,5-, 1,6-, 1,7-, 1,8-, 2,3-, 2,6- oder 2,7- Naphthalinrest), bei dem eine oder zwei CH-Gruppen durch Stickstoffatome ersetzt sein können, und einen divalenten Anthracenrest (z.B. 1,2-, 1,3-, 1,4-, 1,5-, 1,6-, 1,7-, 1,8-, 1,9-, 1,10-, 2,3-, 2,6-, 2,7-, 2,9-, 2,10- oder 9,10- Anthracenrest), bei dem eine oder zwei CH-Gruppen durch Stickstoffatome ersetzt sein können. Bis zu 12 einzelne Wasserstoffatome der $CH_2$-Grup-

pen können jeweils unabhängig voneinander auch an gleichen C-Atomen ersetzt sein durch die Halogene Fluor, Chlor, Brom oder Iod, eine Cyanogruppe oder eine lineare Alkylkette mit bis zu 18 C-Atomen, bei der eine bis 6 $CH_2$-Einheiten unabhängig voneinander ersetzt sein können durch eine Carbonylgruppe, ein Sauerstoffatom, Schwefelatom, Selenatom, Telluratom, eine *cis*- oder *trans*-CH=CH-Gruppe, bei der eine CH-Einheit durch ein Stickstoffatom ersetzt sein kann, eine acetylenische C≡C-Gruppe, einen divalenten Phenylrest (z.B. 1,2-, 1,3- oder 1,4- Phenylrest), divalenten Pyridinrest (z.B. 2,3-, 2,4-, 2,5-, 2,6-, 3,4- oder 3,5- Pyridinrest), divalenten Thiophenrest (z.B. 2,3-, 2,4-, 2,5- oder 3,4-Thiophenrest), divalenten Naphthalinrest (z.B. 1,2-, 1,3-, 1,4-, 1,5-, 1,6-, 1,7-, 1,8-, 2,3-, 2,6- oder 2,7-Naphthalinrest), bei dem eine oder zwei CH-Gruppen durch Stickstoffatome ersetzt sein können, und einen divalenten Anthracenrest (z.B. 1,2-, 1,3-, 1,4-, 1,5-, 1,6-, 1,7-, 1,8-, 1,9-, 1,10-, 2,3-, 2,6-, 2,7-, 2,9-, 2,10- oder 9,10- Anthracenrest), bei dem ein oder zwei CH-Gruppen durch Stickstoffatome ersetzt sein können. Bis zu 12 einzelne Wasserstoffatome der $CH_2$-Gruppen der Alkylreste können jeweils unabhängig voneinander auch an gleichen C-Atomen ersetzt sein durch die Halogene Fluor, Chlor, Brom oder Iod, eine Cyanogruppe oder eine lineare Alkylkette mit bis zu 18 C-Atomen, bei der eine bis 6 $CH_2$-Einheiten unabhängig voneinander ersetzt sein können durch eine Carbonylgruppe, ein Sauerstoffatom, Schwefelatom, Selenatom, Telluratom, eine *cis*- oder *trans*-CH=CH-Gruppe, bei der eine CH-Einheit durch ein Stickstoffatom ersetzt sein kann, eine acetylenische C≡C-Gruppe, einen divalenten Phenylrest (z.B. 1,2-, 1,3- oder 1,4-Phenylreste), divalenten Pyridinrest (z.B. 2,3-, 2,4-, 2,5-, 2,6-, 3,4- oder 3,5- Pyridinrest), divalenten Thiophenrest (z.B. 2,3-, 2,4-, 2,5- oder 3,4- Thiophenrest), divalenten Naphthalinrest (z.B. 1,2-, 1,3-, 1,4-, 1,5-, 1,6-, 1,7-, 1,8-, 2,3-, 2,6- oder 2,7- Naphthalinrest), bei dem eine oder zwei CH-Gruppen durch Stickstoffatome ersetzt sein können, und einem divalenten Anthracenrest (z.B. 1,2-, 1,3-, 1,4-, 1,5-, 1,6-, 1,7-, 1,8-, 1,9-, 1,10-, 2,3-, 2,6-, 2,7-, 2,9-, 2,10- oder 9,10- Anthracenrest), bei dem eine oder zwei CH-Gruppen durch Stickstoffatome ersetzt sein können. $CH_2$-Gruppen, an denen wie vorstehend beschrieben ein Wasserstoffatom ersetzt ist, können auch unter Bildung eines Rings miteinander verknüpft sein, d.h. statt Substituenten zu tragen können die freien Valenzen der Methingruppen bzw. der quartären C-Atome paarweise verknüpft werden, so dass Ringe entstehen, wie z.B. Cyclohexanringe.

Soweit nicht anders angegeben, kann es sich im Rahmen dieser Beschreibung bei einer $CH_2$-Einheit, die definitionsgemäß ersetzt werden kann, auch um eine endständige Einheit in einem Alkylrest/einer Alkylkette, d.h. eine entsprechende Einheit innerhalb einer $-CH_3$ Gruppe handeln. So ist z.B. die auch Bezugnahme auf einen divalenten Ring oder ein divalentes Ringsystem, die eine $CH_2$-Einheit ersetzen können (z.B. divalente Phenylreste oder 1,2-, 1,3- oder 1,4- Phenylreste) hier und im Folgenden so zu verstehen, dass eine der beiden Valenzen auch mit einem H-Atom abgesättigt sein kann. Damit ist auch die Situation umfasst, dass ausgehend von einer Methylgruppe durch Ersetzen einer darin enthaltenen formalen $CH_2$-Einheit ein Phenylrest, Pyridinrest, Thiophenrest, etc. an der entsprechenden Position vorhanden ist. Daher können die Reste $R^1$ bis $R^{11}$ im Rahmen der vorstehenden Definition z.B. auch Arylreste, insbesondere Phenyl- oder Naphtylreste, Heterorarylreste, insbesondere Pyridyl- oder Thiophenylreste, Aralkylreste und Heteroaralkylreste darstellen.

Bevorzugt ist in der Formel (I) der Rest $R^1$ Wasserstoff oder einer der in der allgemeinen Definition genannten Kohlenwasserstoffreste. Besonders bevorzugt ist ein Alkyl, Alkenyl oder Alkinylrest. Dabei sind sowohl lineare Reste als auch verzweigte Reste eingeschlossen, wobei bei den verzweigten Resten wie vorstehend beschrieben ein oder mehrere Wasserstoffatome von $CH_2$ Gruppen durch weitere Alkylketten ersetzt sind, die ebenfalls eine Doppel- oder Dreifachbindung enthalten können. Die Gesamtzahl der Kohlenstoffatome in diesen Alkyl, Alkenyl oder Alkinylresten beträgt bevorzugt 6 bis 20. Besonders bevorzugt ist als $R^1$ ein linearer oder verzweigter Alkylrest gemäß der obigen Definition, der 6 bis 20 C-Atome aufweist.

Bevorzugt sind die Reste $R^2$ bis $R^5$ unabhängig voneinander ausgewählt aus Wasserstoff oder einem der in der allgemeinen Definition genannten Kohlenwasserstoffreste. Besonders bevorzugt sind ein Alkyl, Alkenyl oder Alkinylrest. Dabei sind sowohl lineare Reste als auch verzweigte Reste eingeschlossen, wobei bei den verzweigten Resten wie vorstehend beschrieben ein oder mehrere Wasserstoffatome von $CH_2$ Gruppen durch weitere Alkylketten ersetzt sind, die ebenfalls eine Doppel- oder Dreifachbindung enthalten können. Besonders bevorzugt ist zusätzlich auch ein Arylrest, wie z.B. Phenyl. Die Gesamtzahl der Kohlenstoffatome in diesen Resten beträgt bevorzugt 1 bis 20 für Alkyl, Alkenyl und Alkinylreste, bzw. 6 bis 14 für den Arylrest. Besonders bevorzugt sind $R^2$ bis $R^5$ Wasserstoff oder ein linearer oder verzweigter Alkylrest gemäß der obigen Definition, der 1 bis 10 C-Atome aufweist, insbesondere Wasserstoff.

Bevorzugt ist $R^6$ ausgewählt aus Wasserstoff, einem Alkylrest, einem Arylrest, Heteroarylrest, Aralkylrest oder Heteroaralkylrest. Besonders bevorzugt ist $R^6$ ein Arylrest oder Heteroarylrest, insbesondere ein Arylrest.

Der Alkylrest bzw. der Alkyl-Anteil dieser bevorzugten Reste weist eine lineare Kette von 1 bis 20 C-Atomen auf. Er kann substituiert sein mit einem oder mehreren Substituenten, z.B. einem, zwei oder drei, ausgewählt aus linearen Alkylketten mit bis zu 10 C-Atomen, Cl, Br oder CN, ist jedoch bevorzugt unsubstituiert.

Der Arylrest bzw. der Aryl-Anteil des Aralkylrests ist bevorzugt Phenyl oder Naphtyl, insbesondere Phenyl. Der Heteroarylrest bzw. Heteroaryl-Anteil des Heteroaralkylrests ist bevorzugt Pyridin oder Thiophen.

Bevorzugt ist $R^7$ ausgewählt aus Wasserstoff oder einem Arylrest mit bis zu 10 C-Atomen, Cl, Br oder CN. Besonders bevorzugt ist $R^7$ Wasserstoff.

Bevorzugt sind die Reste $R^8$ bis $R^{11}$ unabhängig voneinander ausgewählt aus Wasserstoff, einem Alkylrest,

einem Alkoxyrest, einem Arylrest, Heteroarylrest, Aralkylrest oder Heteroaralkylrest, Cl, Br oder CN.

Der Alkylrest bzw. der Alkyl-Anteil dieser Reste weist bevorzugt eine lineare Kette von 1 bis 20 C-Atomen auf. Er kann substituiert sein mit einem oder mehreren Substituenten, z.B. einem, zwei oder drei, ausgewählt aus linearen Alkylketten mit bis zu 10 C-Atomen, Cl, Br oder CN, ist jedoch bevorzug unsubstituiert.

Der Arylrest bzw. der 4ryl-Anteil des Aralkylrest ist bevorzugt Phenyl oder Naphtyl. Der Heteroarylrest bzw. Heteroaryl-Anteil des Heteroaralkylrests ist bevorzugt Pyridin oder Thiophen.

Bevorzugt ist mindestens einher der Reste $R^8$ und $R^9$ und mindestens einer der Reste $R^{10}$ und $R^{11}$ Wasserstoff, besonders bevorzugt sind drei der Reste $R^8$ bis $R^{11}$ Wasserstoff. Darüber hinaus ist bevorzugt einer der Reste $R^{10}$ und $R^{11}$ ein Arylrest oder Heteroarylrest, besonders bevorzugt ein Arylrest.

Bevorzugt ist X ausgewählt aus einer bis 12 Methyleneinheiten, wobei eine oder mehrere $CH_2$ Gruppen ersetzt sein können durch eine Gruppe unabhängig ausgewählt aus O, S und Phenylen. Besonders bevorzugt wird X gebildet aus einer oder zwei Phenylen-Gruppen oder einer Bisphenylen-Gruppe in Kombination mit 1 bis 4, bevorzugt 1 oder 2 Methyleneinheiten.

Für die Perylentetracarbonsäurebisimid-Nitrone der allgemeinen Formel (II),

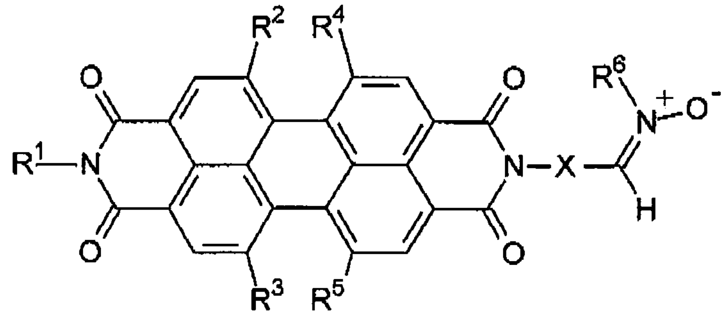

(II)

entsprechen die Definitionen der die Reste $R^1$ bis $R^6$ und X, einschließlich der Definitionen bevorzugte Reste, denjenigen die für die Reste $R^1$ bis $R^6$ und X in Bezug auf die Formel (I) gegeben wurden.

α-Effekt-Verbindungen der allgemeinen Formel (III) können als Elektronendonorgruppen in lichtgetriebenen Systemen zur Ladungstrennung verwendet werden.

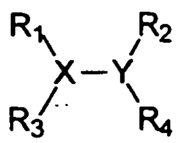

(III)

In Formel (III) sind X und Y gleich oder unterschiedlich und stellen Elemente mit freien, nicht bindenden Elektronenpaaren dar, bevorzugt Elemente der zweiten und dritten Periode, wie Stickstoff, Sauerstoff, Fluor, Schwefel und Chlor, bevorzugt die Elemente der zweiten Periode und von diesen am meisten bevorzugt Stickstoff und Sauerstoff. Die Reste $R_1$ bis $R_4$ sind gleich oder verschieden voneinander und bedeuten unabhängig voneinander Wasserstoff oder lineare Alkylreste mit mindestens einem und höchstens 37 C-Atomen. Es ist für den Fachmann ersichtlich, dass je nachdem, welches Element für X und Y gewählt wird, einer der Reste $R_1$ und $R_3$ bzw. einer der Reste $R_2$ und $R_4$ abwesend sein kann. In dem Alkylrest können eine bis 10 $CH_2$-Einheiten unabhängig voneinander ersetzt sein durch eine Carbonylgruppe, ein Sauerstoffatom, Schwefelatom, Selenatom, Telluratom, eine *cis*- oder *trans*-CH=CH-Gruppe, bei der eine CH-Einheit auch durch ein Stickstoffatom ersetzt sein kann, eine acetylenische C≡C-Gruppe, einen divalenten Phenylrest (z.B. 1,2-, 1,3- oder 1,4- Phenylrest), divalenten Pyridinrest (z.B. 2,3-, 2,4-, 2,5-, 2,6-, 3,4- oder 3,5-Pyridinrest), divalenten Thiophenrest (z.B. 2,3-, 2,4-, 2,5- oder 3,4- Thiophenrest), divalenten Naphthalinrest (z.B. 1,2-, 1,3-, 1,4-, 1,5-, 1,6-, 1,7-, 1,8-, 2,3-, 2,6- oder 2,7- Naphthalinrest), bei dem eine oder zwei CH-Gruppen durch Stickstoffatome ersetzt sein können, und einen divalenten Anthracenrest (z.B. 1,2-, 1,3-, 1,4-, 1,5-, 1,6-, 1,7-, 1,8-, 1,9-, 1,10-, 2,3-, 2,6-, 2,7-, 2,9-, 2,10- oder 9,10- Anthracenrest), bei dem eine oder zwei CH-Gruppen durch Stickstoffatome ersetzt sein können. Bis zu 12 einzelne Wasserstoffatome der $CH_2$-Gruppen können jeweils unabhängig voneinander auch an gleichen C-Atomen ersetzt sein durch die Halogene Fluor, Chlor, Brom oder Iod, eine Cyanogruppe oder eine lineare

Alkylkette mit bis zu 18 C-Atomen, bei der eine bis 6 $CH_2$-Einheiten unabhängig voneinander ersetzt sein können durch eine Carbonylgruppe, ein Sauerstoffatom, Schwefelatom, Selenatom, Telluratom, eine *cis*- oder *trans*-CH=CH-Gruppe, bei der eine CH-Einheit durch ein Stickstoffatom ersetzt sein kann, eine acetylenische C≡C-Gruppe, einen divalenten Phenylrest (z.B. 1,2-, 1,3- oder 1,4-Phenylrest), einen divalenten Pyridinrest (z.B. 2,3-, 2,4-, 2,5-, 2,6-, 3,4- oder 3,5-Pyridinrest), einen divalenten Thiophenrest (z.B. 2,3-, 2,4-, 2,5- oder 3,4- Thiophenrest), einen divalenten Naphthalinrest (z.B. 1,2-, 1,3-, 1,4-, 1,5-, 1,6-, 1,7-, 1,8-, 2,3-, 2,6- oder 2,7- Naphthalinrest), bei dem eine oder zwei CH-Gruppen durch Stickstoffatome ersetzt sein können, und einen divalenten Anthracenrest (z.B. 1,2-, 1,3-, 1,4-, 1,5-, 1,6-, 1,7-, 1,8-, 1,9-, 1,10-, 2,3-, 2,6-, 2,7-, 2,9-, 2,10- oder 9,10- Anthracenrest), bei denen eine oder zwei CH-Gruppen durch Stickstoffatome ersetzt sein können. Bis zu 12 einzelne Wasserstoffatome der $CH_2$-Gruppen in einem Alkylrest können jeweils unabhängig voneinander auch an gleichen C-Atomen ersetzt sein durch die Halogene Fluor, Chlor, Brom oder Iod, eine Cyanogruppe oder eine lineare Alkylkette mit bis zu 18 C-Atomen, bei der eine bis 6 $CH_2$-Einheiten unabhängig voneinander ersetzt sein können durch eine Carbonylgruppe, ein Sauerstoffatom, Schwefelatom, Selenatom, Telluratom, eine *cis*- oder *trans*-CH=CH-Gruppe, bei der eine CH-Einheit durch ein Stickstoffatom ersetzt sein kann, eine acetylenische C≡C-Gruppe, einen divalenten Phenylrest (z.B. 1,2-, 1,3- oder 1,4- Phenylrest), einen divalenten Pyridinrest, (z.B. 2,3-, 2,4-, 2,5-, 2,6-, 3,4- oder 3,5- Pyridinrest), einen divalente Thiophenrest (z.B. 2,3-, 2,4-, 2,5- oder 3,4- Thiophenrest), einen divalenten Naphthalinrest (z.B. 1,2-, 1,3-, 1,4-, 1,5-, 1,6-, 1,7-, 1,8-, 2,3-, 2,6- oder 2,7-Naphthalinrest), bei dem ein oder zwei CH-Gruppen durch Stickstoffatome ersetzt sein können, und einen divalenten Anthracenrest (z.B. 1,2-, 1,3-, 1,4-, 1,5-, 1,6-, 1,7-, 1,8-, 1,9-, 1,10-, 2,3-, 2,6-, 2,7-, 2,9-, 2,10- oder 9,10- Anthracenrest), bei dem eine oder zwei CH-Gruppen durch Stickstoffatome ersetzt sein können. $CH_2$-Gruppen, an denen wie vorstehend beschrieben ein Wasserstoffatom ersetzt ist, können auch unter Bildung eines Rings miteinander verknüpft sein, d.h. statt Substituenten zu tragen, können die freien Valenzen der Methingruppen bzw. der quartären C-Atome paarweise verknüpft werden, so dass Ringe entstehen, wie z.B. Cyclohexanringe. Die Reste $R_1$ bis $R_4$ können außerdem unabhängig voneinander die Halogenatome F, Cl, Br oder I oder CN bedeuten. Bevorzugt sind die Reste $R_1$ und $R_2$ oder die Reste $R_3$ und $R_4$ unter Ringschluss verbunden, so dass sich zusammen mit X und Y ein fünfodcr sechsgliedriger Ring bildet.

Wie bereits für Formel (I) angegeben, kann es sich im Rahmen dieser Beschreibung bei einer $CH_2$ Einheit, die definitionsgemäß ersetzt werden kann, auch um eine endständige Einheit in einem Alkylrest/einer Alkylkette, d.h. eine entsprechende Einheit innerhalb einer $-CH_3$ Gruppe bandeln. Daher können die Reste $R_1$ bis $R_4$ im Rahmen der vorstehenden Definition z.B. auch Arylreste, insbesondere Phenyl- oder Naphtylreste, Heterorarylreste, insbesondere Pyridyl- oder Thiophenykeste, Aralkylreste und Heteroaralkylreste darstellen.

Gewöhnlich erfolgt die beschriebene Verwendung der α-Effekt-Verbindungen der allgemeinen Formel (III) als Elektronendonorgruppen in lichtgetriebenen Systemen zur Ladungstrennung dergestalt, dass die Verbindung über einen beliebigen der Reste $R_1$ bis $R_4$ an eine lichtabsorbierende Einheit gebunden wird, typischerweise über eine kovalente Bindung. Als lichtabsorbierende Einheit, auch als Chromophor bezeichnet, können bekannte Strukturen eingesetzt werden, die in einem für die jeweilige Anwendung geeigneten Spektralbereich absorbieren. Insofern können auch Verbindungen als Systeme zur Ladungstrennung verwendet werden, bestehend aus einer lichtabsorbierenden Einheit, und einer Elektronendonorgruppe die sich formal aus einer Verbindung der Formel (III) ableiten lässt indem von einem beliebigen der Reste $R_1$ bis $R_4$ ein Atom, typischerweise ein H-Atom, abstrahiert wird, so dass der betreffende Rest eine Linkergruppe bildet, die die Verbindung der Formel (III) mit dem Chromophor verbindet.

Desweitern können α-Effekt-Verbindungen der allgemeinen Formel (IIIa) als Elektronendonort gruppen in lichtgetriebenen Systemen zur Ladungstrennung verwendet werden.

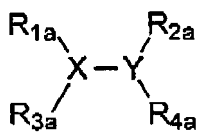

(IIIa)

In Formel (IIIa) sind X und Y gleich oder unterschiedlich und stellen Elemente mit einem freien, nicht bindenden Elektronenpaar dar, ausgewählt aus Stickstoff, Sauerstoff und Schwefiel, bevorzugt aus Stickstoff und Sauerstoff. Ist X Sauerstoff oder Schwefel, so ist $R_{1a}$ abwesend. Ist Y Sauerstoff oder Schwefel, so ist $R_{2a}$ abwesend. Soweit vorhanden, stehen die Reste $R_{1a}$ und $R_{2a}$, für Wasserstoff, einen Alkylrest, insbesondere Methyl, Ethyl, Propyl oder Butyl, oder einen Phenylrest. Die Reste $R_{3a}$ und $R_{4a}$ bilden zusammen mit X und Y einen fünf- oder sechsgliedrigen Ring, der neben den Elementen X und Y als Ringatome Kohlenstoff und optional ein oder mehrere weitere Heteroatome, ausgewählt aus S, N und O umfasst. Der aus $R_{3a}$ und $R_{4a}$ gebildete Ring kann gesättigt sein oder eine Kohlenstoff-Kohlenstoff oder Kohlenstoff-Stickstoff-Doppelbindung umfassen.

Gewöhnlich erfolgt die beschriebene Verwendung der α-Effekt-Verbindungen der allgemeinen Formel (IIIa)

als Elektronendonorgruppen in lichtgetriebenen Systemen zur Ladungstrennung dergestalt, dass die Verbindung über einen beliebigen der Reste $R_{1a}$ bis $R_{4a}$ an eine lichtabsorbierende Einheit gebunden wird, typischerweise über eine kovalente Bindung. Als lichtabsorbierende Einheit, auch als Chromophor bezeichnet, können bekannte Strukturen eingesetzt werden, die in einem für die jeweilige Anwendung geeigneten Spektralbereich absorbieren. Insofern können auch Verbindungen als Systeme zur Ladungstrennung verwendet werden, bestehend aus einer lichtabsorbierenden Einheit, und einer Elektronendonorgruppe die sich formal aus einer Verbindung der Formel (IIIa) ableiten lässt indem von einem beliebigen der Reste $R_{1a}$ bis $R_{4a}$ ein Atom, typischerweise ein H-Atom, abstrahiert wird, so dass der betreffende Rest eine Linkergruppe bildet, die die Verbindung der Formel (IIIa) mit dem Chromophor verbindet.

Insbesondere umfasst die Erfindung auch die Verwendung von Isoxazolidinen der allgemeinen Formel (IV) als Elektronendonorgruppen in lichtgetriebenen Systemen zur Ladungstrennung.

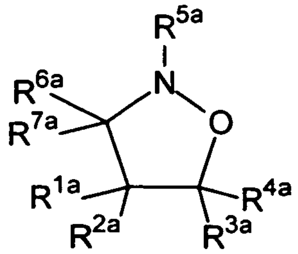

**(IV)**

In der Formel (IV) sind die Reste $R^{1a}$ bis $R^{7a}$ gleich oder verschieden voneinander und bedeuten unabhängig voneinander Wasserstoff oder lineare Alkylreste mit mindestens einem und höchstens 37 C-Atomen. In dem Alkylrest können eine bis 10 $CH_2$-Einheiten unabhängig voneinander ersetzt sein durch eine Carbonylgruppe, ein Sauerstoffatom, Schwefelatom, Selenatom, Telluratom, eine *cis*- oder *trans*-CH=CH-Gruppe, bei der eine CH-Einheit auch durch ein Stickstoffatom ersetzt sein kann, eine acetylenische C≡C-Gruppe, einen divalenten Phenylrest (z.B. 1,2-, 1,3- oder 1,4-Phenylrest), divalenten Pyridinrest (z.B. 2,3-, 2,4-, 2,5-, 2,6-, 3,4- oder 3,5- Pyridinrest), divalenten Thiophenrest (z.B. 2,3-, 2,4-, 2,5- oder 3,4- Thiophenrest), divalenten Naphthalinrest (z.B. 1,2-, 1,3-, 1,4-, 1,5-, 1,6-, 1,7-, 1,8-, 2,3-, 2,6- oder 2,7-Naphthalinrest), bei dem eine oder zwei CH-Gruppen durch Stickstoffatome ersetzt sein können, und einen divalenten Anthracenrest (z.B. 1,2-, 1,3-, 1,4-, 1,5-, 1,6-, 1,7-, 1,8-, 1,9-, 1,10-, 2,3-, 2,6-, 2,7-, 2,9-, 2,10- oder 9,10-Anthracenreste), bei dem eine oder zwei CH-Gruppen durch Stickstoffatome ersetzt sein können. Bis zu 12 einzelne Wasserstoffatome der $CH_2$-Gruppen können jeweils unabhängig voneinander auch an gleichen C-Atomen ersetzt sein durch die Halogene Fluor, Chlor, Brom oder Iod, eine Cyanogruppe oder eine lineare Alkylkette mit bis zu 18 C-Atomen, bei der eine bis 6 $CH_2$-Einheiten unabhängig voneinander ersetzt sein können durch eine Carbonylgruppe, ein Sauerstoffatom, Schwefelatom, Selenatom, Telluratom, eine *cis*- oder *trans*-CH=CH-Gruppe, bei der eine CH-Einheit durch ein Stickstoffatom ersetzt sein kann, eine acetylenische C≡C-Gruppe, einen divalenten Phenylrest (z.B. 1,2-, 1,3- oder 1,4-Phenylrest), einen divalenten Pyridinrest (z.B. 2,3-, 2,4-, 2,5-, 2,6-, 3,4- oder 3,5- Pyridinrest), einen divalenten Thiophenrest (z.B. 2,3-, 2,4-, 2,5- oder 3,4- Thiophenrest), einen divalenten Naphthalinrest (z.B. 1,2-, 1,3-, 1,4-, 1,5-, 1,6-, 1,7-, 1,8-, 2,3-, 2,6- oder 2,7- Naphthalinrest), bei dem eine oder zwei CH-Gruppen durch Stickstoffatome ersetzt sein können, und einen divalenten Anthracenrest (z.B. 1,2-, 1,3-, 1,4-, 1,5-, 1,6-, 1,7-, 1,8-, 1,9-, 1,10-, 2,3-, 2,6-, 2,7-, 2,9-, 2,10- oder 9,10- Anthracenrest), bei denen ein oder zwei CH-Gruppen durch Stickstoffatome ersetzt sein können. Bis zu 12 einzelne Wasserstoffatome der $CH_2$-Gruppen in einem Alkylrest können jeweils unabhängig voneinander auch an gleichen C-Atomen ersetzt sein durch die Halogene Fluor, Chlor, Brom oder Iod, eine Cyanogruppe oder eine lineare Alkylkette mit bis zu 18 C-Atomen, bei der eine bis 6 $CH_2$-Einheiten unabhängig voneinander ersetzt sein können durch eine Carbonylgruppe, ein Sauerstoffatom, Schwefelatom, Selenatom, Telluratom, eine *cis*- oder *trans*-CH=CH-Gruppe, bei der eine CH-Einheit durch ein Stickstoffatom ersetzt sein kann, eine acetylenische C≡C-Gruppe, einen divalenten Phenylrest (z.B. 1,2-, 1,3- oder 1,4-Phenylrest), einen divalenten Pyridinrest (z.B. 2,3-, 2,4-, 2,5-, 2,6-, 3,4- oder 3,5-Pyridinrest), einen divalente Thiophenrest (z.B. 2,3-, 2,4-, 2,5- oder 3,4- Thiophenrest), einen divalenten Naphthalinrest (z.B. 1,2-, 1,3-, 1,4-, 1,5-, 1,6-, 1,7-, 1,8-, 2,3-, 2,6- oder 2,7- Naphthalinrest), bei dem ein oder zwei CH-Gruppen durch Stickstoffatome ersetzt sein können, und einen divalenten Anthracenrest (z.B. 1,2-, 1,3-, 1,4-, 1,5-, 1,6-, 1,7-, 1,8-, 1,9-, 1,10-, 2,3-, 2,6-, 2,7-, 2,9-, 2,10- oder 9,10- Anthracenrest), bei dem eine oder zwei CH-Gruppen durch Stickstoffatome ersetzt sein können. $CH_2$-Gruppen, an denen wie vorstehend beschrieben ein Wasserstoffatom ersetzt ist, können auch unter Bildung eines Rings miteinander verknüpft sein, d.h. statt Substituenten zu tragen, können die freien Valenzen der Methingruppen bzw. der quartären C-Atome paarweise verknüpft werden, so dass Ringe entstehen, wie z.B. Cyclohexanringe. Die Reste $R^{1a}$ bis $R^{4a}$, $R^{6a}$ und $R^{7a}$ können außerdem unabhängig voneinander die Halogenatome F, Cl, Br oder I oder CN bedeuten.

Wie bereits für Formel (I) angegeben, kann es sich im Rahmen dieser Beschreibung bei einer $CH_2$ Einheit, die definitionsgemäß ersetzt werden kann, auch um eine endständige Einheit in einem Alkylrest/einer Alkylkette, d.h. eine entsprechende Einheit innerhalb einer $-CH_3$ Gruppe handeln. Daher können die Reste $R^{1a}$ bis $R^{7a}$ im Rahmen der vorstehenden Definition z.B. auch Arylreste, insbesondere Phenyl- oder Naphtylreste, Heterorarylreste, insbesondere Pyridyl- oder Thiophenylreste, Aralkylreste und Heteroaralkylreste darstellen.

In der Regel erfolgt die beschriebene Verwendung der Isoxazolidine der allgemeinen Formel (IV) als Elektronendonorgruppen in lichtgetriebenen Systemen zur Ladungstrennung dergestalt, dass die Verbindung über einen der Reste $R^{1a}$ bis $R^{7a}$ an eine lichtabsorbierende Einheit gebunden wird, typischerweise über eine kovalente Bindung. Bevorzugt erfolgt diese Bindung über einen der Reste $R^{1a}$ bis $R^{4a}$, $R^{6a}$ oder $R^{7a}$, besonders bevorzugt über den Rest $R^{6a}$. Als lichtabsorbierende Einheit, auch als Chromophor bezeichnet, können bekannte Strukturen eingesetzt werden, die in einem für die jeweilige Anwendung geeigneten Spektralbereich absorbieren. Insofern beinhaltet die Erfindung auch Verbindungen als Systeme zur Ladungstrennung, bestehend aus einer lichtabsorbierenden Einheit, und einer Elektronendonorgruppe die sich formal aus einer Verbindung der Formel (IV) ableiten lässt indem von einem beliebigen der Reste $R^{1a}$ bis $R^{7a}$, bevorzugt von einem der Reste $R^{1a}$ bis $R^{4a}$, $R^{6a}$ oder $R^{7a}$, besonders bevorzugt von Rest $R^{6a}$ ein Atom, typischerweise ein H-Atom, abstrahiert wird, so dass der betreffende Rest eine Linkergruppe bildet, die die Verbindung der Formel (IV) mit dem Chromophor verbindet.

Bevorzugt sind die Reste $R^{1a}$ bis $R^{4a}$ unabhängig voneinander ausgewählt aus Wasserstoff, einem Alkylrest, einem Alkoxyrest, einem Arylrest, Heteroarylrest, Aralkylrest oder Heteroaralkylrest, Cl, Br oder CN. Bevorzugt ist mindestens einer der Reste $R^{1a}$ und $R^{2a}$ und mindestens einer der Reste $R^{3a}$ und $R^{4a}$ Wasserstoff, besonders bevorzugt sind drei der Reste $R^{1a}$ bis $R^{4a}$ Wasserstoff. Darüber hinaus ist bevorzugt einer der Reste $R^{3a}$ und $R^{4a}$ ein Arylrest oder Heteroarylrest, besonders bevorzugt ein Arylrest.

Der Alkylrest bzw. der Alkyl-Anteil dieser Reste weist bevorzugt eine lineare Kette von 1 bis 20 C-Atomen auf. Er kann substituiert sein mit einem oder mehreren Substituenten, z.B. einem, zwei oder drei, ausgewählt aus linearen Alkylketten mit bis zu 10 C-Atomen, Cl, Br oder CN, ist jedoch bevorzugt unsubstituiert.

Der Arylrest bzw. der Aryl-Anteil des Aralkylrests ist bevorzugt Phenyl oder Naphtyl. Der Heteroarylrest bzw. Heteroaryl-Anteil des Heteroaralkylrests ist bevorzugt Pyridin oder Thiophen.

Bevorzugt ist $R^{5a}$ ausgewählt aus Wasserstoff, einem Alkylrest, einem Arylrest, Heteroarylrest, Aralkylrest oder Heteroaralkylrest. Besonders bevorzugt ist $R^5$ ein Arylrest oder Heteroarylrest, insbesondere ein Arylrest.

Der Alkylrest bzw. der Alkyl-Anteil dieser Reste weist bevorzugt eine lineare Kette von 1 bis 20 C-Atomen auf. Er kann substituiert sein mit einem oder mehreren Substituenten, z.B. einem, zwei oder drei, ausgewählt aus linearen Alkylketten mit bis zu 10 C-Atomen, Cl, Br oder CN, ist jedoch bevorzugt unsubstituiert.

Der Arylrest bzw. der Aryl-Anteil des Aralkylrests ist bevorzugt Phenyl oder Naphtyl. Der Heteroarylrest bzw. Heteroaryl-Anteil des Heteroaralkylrests ist bevorzugt Pyridin oder Thiophen.

Bevorzugt sind $R^{68}$ und $R^{7a}$ unabhängig voneinander ausgewählt aus Wasserstoff, einem Alkylrest, einem Alkylrest, bei dem eine oder mehrere $CH_2$ Gruppen ersetzt sind durch eine Gruppe unabhängig ausgewählt aus O, S und Phenylen, einem Alkoxyrest, einem Arylrest, Heteroarylrest, Aralkylrest oder Heteroaralkylrest, Cl, Br oder CN.

Der Alkylrest bzw. der Alkyl-Anteil dieser Reste weist bevorzugt eine lineare Kette von 1 bis 12 C-Atomen auf, wobei die C-Atome wie vorstehend beschrieben ersetzt sein können. Er kann substituiert sein mit einem oder mehreren Substituenten, z.B. einem, zwei oder drei, ausgewählt aus linearen Alkylketten mit bis zu 10 C-Atomen, Cl, Br oder CN, ist jedoch bevorzugt unsubstituiert.

Der Arylrest bzw. der Aryl-Anteil des Aralkylrests ist bevorzugt Phenyl oder Naphtyl. Der Heteroarylrest bzw. Heteroaryl-Anteil des Heteroaralkylrests ist bevorzugt Pyridin oder Thiophen.

Besonders bevorzugt ist $R^6$ ausgewählt aus einem Alkylrest, einem Alkylrest, bei dem eine oder mehrere $CH_2$ Gruppen ersetzt sind durch Phenylen oder Bisphenylen, einem Arylrest, Heteroarylrest, Aralkylrest oder Heteroaralkylrest und $R^{7a}$ ist bevorzugt Wasserstoff.

Wie oben erläutert, eignet sich $R^{6a}$ besonders als Linkergruppe, mit deren Hilfe die Verbindung der Formel (IV) mit einem Chromophor verknüpft werden kann. In diesem Fall ist der Rest $R^{6a}$ ein zweiwertiger Rest, der formal durch Abstraktion eines H-Atoms von den vorstehend genannten Resten erhalten wird

Zur Darstellung einer Perylenbisimid-Dyade der Formel (I) kann ein Olefin in einer 1,3-dipolaren Cycloaddition aus einem Nitron der Formel (II) und einem Olefin synthetisiert werden. Nitrone der Formel (II) können ausgehend von einem Perylen-3,4:9,10-tetracarbonsäurebisimid synthetisiert werden, das eine Aldehydfunktion trägt, die über die Gruppe -X- an ein Imid-Stickstoffatom gekoppelt ist (H. Langhals et al., Eur. J. Org. Chem., 2007, 4328-4336). Der Aldehyd wird mit einem Hydroxylaminderivat umgesetzt, z.B. N-Methylhydroxylamin uns N-Phenylhydroxylamin, wodurch das Nitron der Formel (III) erhalten wird.

Beispielhaft wird die Synthese von Verbindungen der Formel (I) und (II) in den Figuren 1 und 2 gezeigt. Als Ausgangsmaterial wird in Figur 1 der Aldehyd **2** (H. Langhals et al., Eur. J. Org. Chem. 2007, 4328-4336) eingesetzt und dieser mit *N*-Methylhydroxylamin zum Methylnitron **3** umgesetzt. Bei **3** ist die Fluoreszenz gelöscht, so dass die Verbindung bereits formal die Bedingungen für die lichtgetriebene Ladungstrennung erfüllt, allerdings ist Stabilität geringer als die der Verbindungen der Formel (I). Mit dem 1,3-Dipol **3** gelang keine Umsetzung mit Styrol. Jedoch kann durch die Umsetzung von **2** mit Phenylhydroxylamin das reaktionsfähigere *N*-Phenylderivat **4** synthetisiert werden, das glatt mit einem Überschuss an Styrol zu **5** reagiert, das als Diastereomerenpaar **5a** und **5b** erhalten wird. Als weitere Beispiele wurden die Olefine Methylmethacrylat und Crotonsäuremethylester zu den Isoxazolidinen **6** und **7** umgesetzt, bei denen jeweils ein Diastereomer erhalten wurde, siehe Figur 2. Auch Acrylnitril reagiert glatt mit **4,** und bildet das Regioisomerenpaar **8** und **9.**

Figur 3 zeigt beispielhaft die Synthese einer Verbindung der Formel (I) und der Formel (II), in der der Phenylspacer durch einen Biphenylspacer ersetzt ist. Eine zu **3** analoge Umsetzung von **10** mit *N*-Methylhydroxylamin zum Nitron **11** war problemlos möglich. Die Reaktivität des Nitrons ist allerdings so klein, dass bei seiner Umsetzung mit Styrol seine Zersetzungsreaktionen im Vordergrund stehen. Deshalb wurde der Aldehyd **10** in Gegenwart von Styrol mit *N*-Phenylhydroxylamin umgesetzt, so dass das entstehende Nitron direkt abgefangen wurde. Auf diesem Weg kann das Isoxazolidin **12** als Diastereomerenpaar **12a** und **12b** erhalten werden. Es wird mit außerordentlich ähnlichen Eigenschaften der beiden Diastereomeren gerechnet, so dass eine Auftrennung für die ganz überwiegende Zahl der Anwendungen nicht erforderlich ist.

Die UV/Vis-Absorptionsspektren der Verbindungen der getesteten Verbindungen der Formeln (I) und (II) sind im sichtbaren Spektralbereich identisch und weichen nur um ein halbes nm vom Spektrum des Farbstoffe **1a** ab, der zwei *sec*-Alkylgruppen an den Stickstoffatomen trägt. Im UV-Bereich sind die Unterschiede etwas ausgeprägter; siehe Figur 5. So trägt der Biphenylspacer unterhalb von 300 nm deutlich zur Absorption bei. Die Unterschiede in den Spektren der anderen Derivate sind auch in diesem Spektralbereich wenig ausgeprägt, so dass die Substanzen von den Spektren her austauschbar sind. Da die Spektren der diversen Derivate praktisch identisch sind, kann man von einer elektronischen Entkopplung von Chromophor und funktionaler Einheit ausgehen, weil sich andernfalls eine Rückwirkung ergeben müsste.

Die Fluoreszenz der Verbindungen der Formel (I) ist überraschenderweise gelöscht. Die Effizienz dieser Löschung ist in Anbetracht der erwähnten starken Fluoreszenz der Amin-Derivate erstaunlich und bemerkenswert. Als mögliche Erklärung kann die Fluoreszenzlöschung auf eine Elektronenübertragung von der Isoxazolidin-Einheit, die durch den $\alpha$-effekt elektronenreich ist, auf die optisch angeregte Perylen-Einheit zurückgeführt werden; siehe Figur 4. Das HOMO des elektronischen Grundzustand, das durch die optische Anregung nur noch halb gefiillt ist, wird durch die Elektronenübertragung wieder vollständig gefällt. Eine Rückkehr des angeregten Elektrons unter Lichtabgabe in sein ursprüngliches Orbital wird damit unterbunden und die Fluoreszenz gequencht. Dabei wird nicht nur die Fluoreszenz der Verbindungen mit einem einfachen Phenyl-Spacer zwischen Chromophor und der Isoxazolidin-Einheit gelöscht, sondern auch bei Verbindungen in denen längere Spacer-Gruppierungen wie ein BiphenylSpacer vorliegen. Hier ist der Abstand zwischen Elektronendonor und Elektronenacceptor bereits wesentlich größer und damit erfolgt die Ladungstrennung über eine erhebliche Distanz. Die Elektronenübertragung erfolgt also sehr effizient.

Die Fluoreszenzquenchung bei den Verbindungen der Formel (I) durch Elektronenübertragung kann experimentell eindeutig von Prädissoziationsprozessen als alternative Möglichkeit unterschieden werden. Wenn man beispielsweise das Isoxazolidin **5** (Fig. 1) mit Trifluoressigsäure protoniert oder mit Bortrifluorid-Etherat komplexiert, wird die $\alpha$-Effekt-Wechselwirkung durch Blockieren des freien Elektronenpaars am Stickstoffatom aufgehoben und darüber hinaus auch noch die Lage der nichtbindenden Orbitale des daran geknüpften Sauerstoffatoms abgesenkt; die Festigkeit der N-O-Bindung ändert sich dagegen durch die Protonierung nur unbedeutend. Eine solche Protonierung schaltet die Fluoreszenz des Farbstoffs voll ein, so dass dies eine durch den $\alpha$-Effekt begünstigte Elektronenübertragung eindeutig belegt. Dementsprechend können die Isoxazolidine als Basen mit einem $pK_a$ von 5 bis 6 (H. Langhals, T. Becherer, J. Lindner, A. Obermeier, Eur. J. Org. Chem., 2007, 26, 4328-4336) sogar als Fluoreszenzindikator verwendet werden.

Die lichtgetriebene Ladungstrennung in den erfindungsgemäßen Verbindungen ist für technische Anwendungen ausgesprochen interessant, denn Ladungstrennungen spielen nicht nur im Photosynthese-Reaktionszentrum eine zentrale Rolle, sondern auch bei Systemen zur photovoltaischen Energiegewinnung. Der verhältnismäßig große Abstand zwischen den getrennten Ladungen ist günstig für einen weiteren Transport zur Nutzung, denn er erschwert eine Rekombination, die die absorbierte Energie in Wärme umwandeln würde.

Für eine technische Nutzung in der Photovoltaik kann man z.B. erfindungsgemäße Verbindungen der Formel (I) direkt in Schichtsysteme einbauen. Noch geschickter ist es, die ladungstrennende Struktur auf einer Oberfläche zu fixieren, beispielsweise mit Hilfe der 1,3-dipolaren Cycloaddition. Hier kann man beispielsweise eine Metalloberfläche, die leicht Elektronen abgibt, wie z.B. Aluminium, Magnesium oder Calcium, mit einem Styrolderivat belegen, das eine Ankergruppe trägt, wie z.B. eine Carboxylat-Gruppe. Führt man jetzt die 1,3-dipolare Cycloaddition mit einem Perylen-Nitron de Formel (II), wie z.B. **4** durch, dann befindet sich das dadurch gebildete Isoxazolidin direkt auf der Metalloberfläche (K. Rueck-Braun, T. E. H. Freysoldt, F. Wierschem, Chem. Soc. Revs. 2005, 34, 507-516). Als Alternative kann man auch solche Isoxazolidine mit Ankergruppen konventionell aufbauen und dann mit einer Metalloberfläche in Kontakt bringen, so dass sie dort gebunden werden. Wenn man die Metalloberfläche mit einem mehr lipophilen Medium in Kontakt bringt, dann kann man eine verstärkte Wechselwirkung mit diesem Medium rechnen. Im Idealfall richten sich dann die über Isoxazolidine gebundenen Chromophore auf und ragen in das Medium. Man erreicht damit eine optimale

Ladungstrennung von der Metalloberfläche weg, und ein elektrisch leitendes Medium kann dann die Ladung des Perylen-Chromophors übernehmen. Ein solches photovöltaisches System ist beispielsweise deshalb von besonderem Interesse, weil es im lichtabsorbierenden Bereich ausschließlich aus Wasserstoff, Kohlenstoff, Sauerstoff und Stickstoff bestehen und daher nach der Verwendung problemlos entsorgt werden kann. Die als lichtabsorbierende Struktur verwendeten Perylenfarbstoffe sind außerordentlich lichtecht - sie gehören zu den lichtechtesten Fluoreszenzfarbstoffen überhaupt - so dass eine sehr lange Lebensdauer des Systems erreicht werden kann. Da es sich bei den neuen Farbstoff-Systemen der Formeln (I) und (II) um lösliche Substanzen handelt, können sie aus Lösung auf die Oberflächen aufgebracht werden. Dies kann z.B. durch Gießen, Spincoaten oder aber auch über Tintenstrahldruck geschehen. Mit dem letzteren Verfahren hat man außerdem die Möglichkeit, die Belegung der Oberfläche in Mikrometer-Dimensionen zu strukturieren. Dies kann z.B. von Vorteil sein, wenn der Photovoltaik-Strom von einzelnen kleinen Bereichen als einzelnen Solarzellen oder von Gruppen solcher Solarzellen abgeführt werden soll. Dies kann z.B. den Vorteil haben, dass man einzelne Zellen der Gruppen abschalten kann, wenn diese z.B. defekt worden sind, ohne die anderen zu beeinträchtigen. Von besonderem Vorteil ist auch die Flexibilität der organischen Materialien, mit denen die Solarzellenanlagen an gekrümmte Oberflächen, wie z.B. konventionelle Dachziegel angepasst werden können; dies ist mit den jetzt überwiegend benutzen, ausgesprochen spröden anorganischen Materialien kaum möglich.

Man kann auch die lichtgetriebene Ladungstrennung durch die neuen Farbstoffe der Formeln (I) und (II) in den bekannten Titandioxid-Solarzellen einsetzen, von denen die Grätzel-Zelle die bekannteste ist. Dieser Solarzellen-Typ setzt in der Regel einen Elektrolyt als Medium ein, so z.B. konventionelle flüssige Elektrolyte wie mineralsalzhaltige Flüssigkeiten, organische flüssige Elektrolyte, wie diverse Imidazolium-Salze, oder auch feste Elektrolyte. Bei diesen Zellen besteht das Problem, dass das Titandioxid nicht im sichtbaren Bereich, sondern im UV-Bereich absorbiert, so dass nur eine geringe Energieausbeute bei Bestrahlung mit Sonnenlicht resultiert. Hier werden Hilfsstoffe benötigt, sie im längerwelligen Bereich absorbieren und dort die Ladungstrennung bewerkstelligen. Die neuen Verbindungen der Formeln (I) und (II) sind für solche Anwendungen prädestiniert.

Schließlich kann man die neuen Verbindungen der Formeln (I) und (II) auch mit konventionellen Silicium-Solarzellen kombinieren und auf diese Weise Mehrschicht-Solarzellen mit organischem Material erzeugen. Das organische Material kann dabei für eine effiziente lichtgetriebene Ladungsträgerinjektion in das Silicium verwendet werden. Der erreichbare hohe molare Absorptionskoeffizient und das problemlose Anpassen an diverse Spektralbereiche ist ein besonderer Vorteil einer Beschichtung mit organischem Material. Hierbei ist insbesondere zu bemerken, dass die organischen Materialien unproblematisch in der Herstellung, Handhabung und Entsorgung sind. Es kann für ein Recycling sogar von der Silicium-Oberfläche entfernt werden, so dass reines Silicium zurückgewonnen werden kann.

Außer in der Photovoltaik kann man die Ladungstrennung auch für chemische Reaktionen einsetzen. So kann man z.B. mit den lichtinduzierten Radikalanionen der Perylenbisimide der Formel (I) chemische Reduktionen erzielen; hierfür kann man z.B. die komplementär gebildete positive Ladung der Isoxazolidine über Elektroden oder andere Einrichtungen abführen. In gleichem Maße kann man die Isoxazolidin-Strukturen der Formel (I) als Oxydationsmittel verwenden. Auf diese Weise gelingt es z.B. aus Solarstrahlung chemische Prozessenergie zu gewinnen.

Die vorliegende Erfindung umfasst daher auch die Verwendung der hier beschriebenen Substanzen, insbesondere Verbindungen der Formel (I) und (II) zur Gewinnung von Solarenergie, typischerweise in photovoltaischen Zellen, bzw. eine photovoltaische Zelle, die eine der hier beschriebenen Verbindungen enthält. Als Beispiel kann insbesondere eine Titandioxid-Zelle, wie die Grätzel-Zelle, genannt werden

Sie umfasst auch die die Verwendung der hier beschriebenen Substanzen, insbesondere Verbindungen der Formel (I) und (II) als lichtgetriebene Reduktionsmittel oder Oxidationsmittel für chemische Reaktionen.

Sie umfasst auch die die Verwendung der hier beschriebenen Substanzen, insbesondere Verbindungen der Formel (I) und (II), als Fluoreszenzindikatoren, z.B. für Protonensäuren, bevorzugt Mineralsäuren wie Schwefelsäure, Salzsäure, Salpetersäure und Phosphorsäure oder für Lewis-Säuren, wird Zinkchlorid, wasserfreies Einen(II)chlorid, wasserfreies Eisen(III)chlorid, wasserfreies Aluniniumchlorid.

Insbesondere die Nitrone der Formel (II) können darüber hinaus auch als Indikator für Olefine eingesetzt werden, da sie mit diesen über eine 1,3 dipolare Cycloaddition reagieren können. Insofern ist auch ein Verfahren zum Nachweis der Olefine Gegenstand der Erfindung, umfassend das Kontaktieren einer Probe, die auf das Vorliegen eines Olefins überprüft werden soll, mit einem Nitron der Formel (II). Bevorzugte Olefine sind ungesättigte Fettsäuren wie Ölsäure, Linolsäure und Linolensäure oder auch deren trans-Isomere, die, als *trans*-Fettsäuren bekannt sind. Das Verfahren kann z.B. auch zur Unterscheidung von cis-Fettsäuren von trans-Fettsäuren eingesetzt werden.

Weitere Einsatzgebiete liegen für die Verbindungen der Formel (I) generell auf Gebieten, in denen Perylenfarbstoffe Anwendung finden. Beispielhaft genannt sei der Einsatz in Datenspeichern, bevorzugt in optischen Speichern, beispiele sind Systeme wie die CD- oder DVD-Platten, der Einsatz in OLEDS (organischen Leuchtdioden), der Einsatz als Pigmente für Leimfarben und verwandten Farben wie Aquarell-Farben und Wasserfarben und Farben für Tintenstrahldrucker Papierfarben, Druckfarben, Tinten und Tuschen und andere Farben für Mal- und Schreib-Zwecke und in Anstrichstoffen, als Pigmente für Lacke, bevorzugte Lacke sind Kunstharz Lacke wie Acryl- oder Vinyl-Harze, Polyesterlacke, Novolacke, Nitrocellulose-Lacke (Nitrolacke) oder auch Naturstoffe wie Zaponlack, Schellack oder Qi-Lack (Japanlack bzw. Chinalack oder ostasiatischer Lack), zur Masse-Färbung von Polymeren, zur Färbung von Naturstoffen, als Beizenfarbstoffe, z.B. zur Färbung von Naturstoffen, als Farbmittel, z.B. zur Färbung von Farben, Lacken und anderen Anstrichsstoffen, Papierfarben, Druckfarben, Tinten und andere Farben für Mal- und Schreib-Zwecke, als Pigmente in der Elektrophotographie oder der Einsatz für Sicherheitsmarkierungs-Zwecke, bevorzugt für Schecks, Scheckkarten, Geldscheine Coupons, Dokumente, Ausweispapiere und dergleichen.

**Beispiele**

**Allgemeines.** IR-Spektren: Perkin Elmer 1420 Ratio Recording Infrared Spektrometer, FT 1000; UV/Vis-Spektren: Varian Cary 5000 und Bruins Omega 20; Fluoreszenzspektren: Perkin Elmer FS 3000 (totalkorrigiert); NMR-Spektroskopie: Varian Vnmrs 600 (600 MHz); Massenspektrometrie: Finnigan MAT 95.

***N*-(1-Hexylheptyl)-*N'*-(4-N"-methylcarbaldimin-*N*"-oxidobenzyl)perylen-3,4:9,10-tetracarbonsäure-bisimid (3):** *N*-(4-Formylbenzyl)-*N'*-(1-hexylheptyl)perylen-3,4:9,10-bis (dicarboximid) (2 erhalten entsprechend H. Langhals, T. Becherer, J. Lindner, A. Obermeier, Eur. J. Org. Chem. 2007, 4328-4336, 1.10 g, 1.59 mmol), *N*-Methylhydroxylamin-Hydrochlorid (200 mg, 2.39 mmol) und $NaHCO_3$ (287 mg, 3.31 mmol) wurden in $CH_2Cl_2$ (50 mL) gelöst, mit $MgSO_4$ (400 mg) versetzt, 5 h unter Rückfluss erhitzt, 16 h bei Raumtemperatur gerührt, filtriert, eingedampft und säulenchromatographisch gereinigt (Kisselgel, Dichlormethan/Methanol 30:1). Ausb. 830 mg (65 %) roter Farbstoff, Schmp. >300°C. $R_f$ (Kieselgel, Dichlormethan/Methanol 25:1) = 0.30. IR (ATR): $\tilde{\nu}$ = 2955.4 m, 2919.5 s, 2854.0 m, 1692.4 s, 1647.1 s, 1591.7 s, 1574.5 m, 1505.8 w, 1465.8 w, 1435.8 m, 1402.5 m, 1353.1 m, 1337.1 s, 1250.4 m, 1171.9 m, 1127.2 w, 1109.4 w, 1005.5 w, 982.7 w, 851.2 w, 809.2 s, 744.5 m, 645.2 w $cm^{-1}$. $^1$H-NMR (600 MHz, $CDCl_3$, 25°C): $\delta$ = 0.83 (t, 1 H, $^3J$ = 6.8 Hz), 1.24-1.40 (m, 16 H, $CH_2$), 1.88-1.93 (m, 2 H, $\beta$-$CH_2$), 2.24-2.30 (m, 2 H, $\beta$-$CH_2$), 3.88 (s, 3 H, $CH_3$), 5.16-5.21 (m, 1 H, CH-N), 5.42 (s, 2 H, $CH_2$-N), 7.33 (s, 1 H, CH-N), 7.60 (d, 1 H, $^3J$=7.1 Hz), 8.17 (d, 1 H, $^3J$=7.1 Hz), 8.57-8.68 ppm (m, 8 H, $H_{pery}$). $^{13}$C-NMR (150 MHz, $CDCl_3$, 25°C): $\delta$ = 14.3, 22.8, 27.2, 29.4, 32.0, 32.6,43.8, 55.1, 1.23.1, 123.4, 126.5, 126.6, 128.9, 129.2, 129.6, 129.7, 130.0, 130.2, 131.3, 131.8, 132.0, 134.4, 135.1, 139.6, 163.5 ppm.. UV/Vis ($CHCl_3$): $\lambda_{max}$ ($E_{rel}$) = 529 (1.0), 492 (0.60), 463 nm (0.21). MS (DEI$^+$ / 70 eV): *m/z* (%) = 720 (7) [$M^+$], 719 (14), 704 (17), 703 (38), 702 (24), 538 (15), 537 (19), 523 (22), 522 (64), 521 (100), 520 (21), 509 (14), 508 (21), 374 (14), 373 (26), 346 (18), 260 (10), 148 (13). $C_{46}H_{45}N_3N_3O_5$ (719.9): Ber. C 76.75, H 6.30, N 5.84; Gef. C 76.69, H 6.24, N 5.66.

***N*-(1-Hexylheptyl)-*N'*-(4-*N*"-phenylcarbaldimin-*N*"-oxedobenzyl)perylen-3,4:9,10-tetracarbonsäure-bisimid (4):** Formylbenzyl)-*N*-(1-hexylheptyl)perylen-3,4:9,10-bis(dicarboximid) (2, erhalten entsprechend. H. Langhals, T. Becherer, J. Lindner, A. Obermeier, Eur. J. Org. Chem. 2007, 4328-4336 1.10 g, 1.59 mmol) und *N*-Phenylhydroxylamin (200 mg, 1.83 mmol) wurden in $CH_2Cl_2$ (50 mL) gelöst mit $MgSO_4$ (400 mg) versetzt, 5 h unter Rückfluss erhitzt, 16 h bei Raumtemperatur gerührt, filtriert, eingedampft, und säulenchromatographisch gereinigt (Kieselgel, Dichlormethan/Methanol 30:1). Ausb. 1.15 g (93 %) roter Farbstoff, Schmp. >300°C. $R_f$(Kieselgel, Dichlormethan/Methanol 25:1) = 0.36. IR (ATR): $\tilde{\nu}$ = 3066.9 w, 2952.0 m, 2923.4 s, 2854.1 m, 1691.8 s, 1646.1 s, 1592.0 s, 1575.4 m, 1504.9 w, 1483.0 w, 1457.9 w, 1435.6 w, 1.402.6 m, 1.334.8 s, 1249.0 m, 1170.8 m, 1125.9 w, 1108.1 w, 1069.6 w, 1022.9 w, 987.3 w, 892.3 w, 850.5 w, 832.3 w, 809.6 m, 763.0 w, 684.9 w, 660.6 w, 622.5 w $cm^{-1}$. $^1$H-NMR (600 MHz, $CDCl_3$, 25°C): $\delta$ = 0.83 (t, 1 H, $^3J$=6.8 Hz, 6 H), 1.24-1.40 (m, 16 H, $CH_2$), 1.88-1.93 (m, 2 H, $\beta$-$CH_2$), 2.24-2.30 (m, 2 H, $\beta$-$CH_2$), 5.16-5.21 (m, 1 H, CH-N), 5.37 (s, 2 H, $CH_2$-N), 7.43-7.46 (m, 3 H, $H_{aryl}$), 7.66 (d, 1 H, $^3J$=8.4 Hz, $H_{aryl}$), 7.74 (d, 1 H, $^3J$=6.7 Hz, $H_{aryl}$), 7.90 (s, 1 H, CH-N), 8.24-8.36 ppm (m, 8 H, $H_{pery}$). $^{13}$C-NMR (150 MHz, $CDCl_3$, 25°C): $\delta$ = 14.0, 27.0, 29.2, 31.8, 32.4, 43.5, 54.9, 121.7, 122.7, 122.9, 123.3, 124.1, 125.9, 126.0, 129.0, 129.1, 129.2, 129.3, 129.3, 129.9, 130.1, 130.8, 131.2, 131.6, 133.8, 134.0, 134.4, 139.8, 149.0, 163.0, 163.3 ppm. UV/Vis ($CHCl_3$): $\lambda_{max}$ ($\varepsilon$) = 463 (19600), 492 (53400); 529 nm (84300). MS (EI$^+$ / 70 eV): *m/z* (%) = 782 (10) [$M^+$ + H], 767 (15), 766 (33), 690 (26), 584 (56), 583 (87), 509 (50), 508 (90), 374 (14), 346 (21), 285 (23), 284 (100), 210 (20), 209 (25), 208 (24). $C_{40}H_{41}N_7O_4$ (683.8): Ber. C 70.26, H 6.04, N 14.34; Gef. C 69.96, H 5.89; N 14.15;

***N*-(1-Hexylheptyl)-*N'*-(4-N"-metbylcarbaldimin-*N*"-oxidobiphenyl)perylen-3,4:9,10-tetracarbonsäure-bisimid (11):** *N*-(4-Carboaldehydbiphenyl-4'-methyl)-*N'*-(1-hexylheptyl)perylen-3,4:9,10-tetracarbonsäurebisimid (**10**, 100 mg, 0.130 mmol) wurde in Styrol (5 mL) gelöst, mit *N*-Methylhydroxylamin-hydrochlorid (109 mg, 1.30 mmol) und Natriumhydrogencarbonat (109 mg, 1.30 mmol) versetzt und auf 85°C erhitzt (leichte Gasentwicklung). Der nach 30 Minuten ausgefallene rote Feststoff wurde abfiltriert und säulenchromatographisch gereinigt (Kieselgel, Dichlormethan/Methanol 30:1). Zwei Nebenprodukte und das Ausgangsmaterial werden rasch eluiert und dann das Reaktionsprodukt als rote Bande. Diese wurde eingedampft, in wenig Dichlormethan gelöst, mit Methanol gefällt, abfiltriert und bei Raumtemperatur getrocknet. Ausb. 53 mg (51 %) roter Feststoff, Schmp. > 300 °C. $R_f$ (Kieselgel, Dichlormethan/Methanol 30:1) = 0.14. IR (ATR): $\tilde{\nu}$ = 2953.0 m, 2924.3 m, 2855.7 m, 1691.8 s, 1651.9 s, 1592.9 s, 1576.8 m, 1507.4 w, 1495.9 w, 1456.9 w, 1435.7 m, 1403.5 m, 1379.0 w, 1333.6 s, 1248.8 m, 1218.2 w, 1195.3 w, 1169.2 m, 1126.5 m, 1106.7 m, 1003.1 w, 988.4 w, 943.0 w, 853.3 m, 808.0 s, 782.8 m, 748.1 s, 721.2 m, 667.0 w, 639.5 m, 615.4 m, 587.4 m $cm^{-1}$. $^1$H-NMR (600 MHz, $CDCl_3$, 25°C): $\delta$ = 0.82 (t, $^3J_{H,H}$ = 7.0 Hz, 6 H, $CH_3$), 1.22-1.36 (m, 16 H, $CH_2$), 1.85-1.91 (m, 2 H, $\beta$-$CH_2$), 2.22-2.29 (m, 2 H, $\beta$-$CH_2$), 3.89 (s, 3 H, $CH_3$), 5.16-5.21 (tt, $^3J_{H,H}$ = 5.9 Hz, $^3J_{H,H}$= 9.3 Hz, 1 H, $\alpha$-CH), 5.42 (s, 2

H, $CH_2$-N), 7.38 (s, 1 H, CH=N), 7.54-7.61 (m, 8 H, $H_{arom}$), 8.48-8.54 ppm (m, 8H, $H_{perylen}$). $^{13}$C-NMR (150 MHz, $CDCl_3$, 25°C): $\delta$ = 14.0, 22.6, 27.0, 29.2, 29.9, 31.8, 32.4, 43.4, 54.9, 122.9, 123.0, 123.1, 123.2, 127.0, 127.2, 127.5, 127.6, 128.9, 129.3, 129.5, 129.6, 129.7, 129.7, 130.2, 131.6, 131.6, 134.1, 134.8, 134.9, 135.0, 135.2, 136.7, 137.4, 139.0, 139.5, 163.3, 163.4 ppm. UV/Vis ($CHCl_3$): $\lambda_{max}$ ($E_{rel}$) = 463 (0.22), 492 (0.61), 529 nm (1.0). HRMS ($C_{52}H_{49}N_3O_5$): Ber. *m*/*z*: 795.369, Gef. *m*/*z*: 795.371, $\Delta$ = 2 mmu.

***N*-(1-Hexylheptyl)-*N'*-4-(5-methyloxycarbonyl-5-methyl-2-phenylisoxazolidin-3-yl)benzylperylen-3,4: 9,10-tetracarbonsäurebisimid (6):** *N*-(1-Hexylheptyl)-*N'*-(4-*N*" - phenylcarbaldimin-N"-oxidobenzyl)perylen-3,4:9,10-tetracarbonsäure-bisimid (4, 110 mg, 141 $\mu$mol) wurden in Methmethylacrylat (20 mL) suspendiert, auf 60°C erhitzt (nach 3 h vollständige Lösung) abkühlen lassen, im Vakuum eingedampft und chromatographisch gereinigt (Kieselgel, $CH_2Cl_2$/MeOH 50:1). Ausb. 87 mg (70%), Schmp.> 300°C. $R_f$ (Kieselgel, $CH_2Cl_2$/MeOH 50:1) = 0.80. IR (ATR): $\tilde{\nu}$ = 2921 m, 2852 m, 1737 w, 1693 s, 1654 s, 1593 s, 1577 m, 1507 w, 1486 w, 1434 w, 1403 m, 1377 w, 1332 s, 1300 w, 1248 w, 1201 w, 1170 w, 1125 w, 1101 w, 1021w, 981 w, 853 w, 809 m, 743 w, 694 w $cm^{-1}$. $^1$H-NMR (600 MHz, $CDCl_3$, 25°C): $\delta$ = 0.82 (t, 3 H, $^3$=*J*=7.0 Hz, $CH_3$), 1.19 - 1.38 (m, 16 H, $CH_2$), 1.61 (s, 3 H, $CH_3$), 1.85 - 1.91 (m, 2 H, $\beta$-$CH_2$), 2.22 - 2.29 (m, 2 H, $\beta$-$CH_2$), 2.27 (dd, 1H, $^3J$ = 7.4 Hz, $^2J$ = 12.5 Hz), 3.31 (dd, 1 H, $^3J$ = 8.9 Hz, $^2J$ = 12.5 Hz), 3.55 (s, 3 H, $OCH_3$), 5.16 - 5.21 (m, 1 H, $\alpha$-CH), 4.74 (dd, 1 H, $^3J$ = 7.4 Hz, $^3J$ = 8.9 Hz), 5.39 (s, 2 H, N-$CH_2$), 6.81 - 6.83 (m, 1 H, $H_{aryl}$), 6.89 - 6.90 (m, 2 H, $H_{aryl}$), 7.18 - 7.13 (m, 2 H, $H_{aryl}$),7.57 (d, 2 H, $^3J$=8.4 Hz), 7.43 (d, 2H, $^3J$=8.4 Hz), 8.53 (d, 2 H, $^3J$=8.1 Hz, $H_{perylen}$), 8.56 (d, 2 H, $^3J$=8.1 Hz, $H_{perylen}$), 8.62 - 8.67 ppm (m, 4 H, $H_{perylen}$). $^{13}$C-NMR (150 MHz, $CDCl_3$, 25°C): $\delta$ = 14.0, 22.5, 26.9, 29.2, 29.7, 31.8, 32.4, 43.4, 43.4, 49.8, 52.3, 54.8, 69.4, 83.2, 114.5, 117.0, 121.4, 122.9, 123.2, 124.1, 126.3, 126.6, 128.4, 129.6, 131.0, 131.0, 131.6, 134.2, 134.9, 136.4, 140.8, 151.1, 163.4, 164.6, 173.4 ppm. UV/Vis ($CHCl_3$): $\lambda_{max}$ ($\varepsilon$) = 459.1 (18600), 491.0 (51400), 527.4 nm (85800). MS (DEI$^+$/70 eV): *m*/*z* (%): 690 (33) [$M^+$], 508 (100) [$M^+$ - $C_{13}H_{26}$], 374 (14), [$M^+$- $C_{21}H_{35}O_2$], 346 (19) [$M^+$ - $C_{22}H_{34}NO_2$], 44 (15) [$CH_2NO$]. HRMS ($C_{56}H_{55}N_3O_7$): Ber. *m*/*z* 690.309, Gef. *m*/*z* 690.308, $\Delta$ = 1 mmu. $C_{56}H_{55}N_3O_7$ (882.1): Ber. C 76.25, H 6.09, N 4.76; Gef. C 75.88, H 6.38, N 4.59.

***N*-(1-Hexylheptyl)-*N'*-4-(5-phenyl-2-phenylisoxazolidin-3-yl)benzylperylen-3,4:9,10-tetracarbonsäure-bisimid (5):** *N*-(1-Hexylheptyl)-*N'*-(4-N"-phenylcarbaldimin-*N*"-oxidobenzyl)perylen-3,4:9,10-tetracarbonsäurebisimid (4, 120 mg, 153 $\mu$mol) wurden in Styrol (10 mL) gelöst, auf 85°C erhitzt, nach 1 h abgekühlt, im Vakuum eingedampft und chromatographiert (Kieselgel, $CH_2Cl_2$/MeOH 20:1, Mischung aus zwei Diastereomeren, de = 77%). Ausb. 102 mg (75%), Schmp. > 300°C. $R_f$ (Kieselgel, $CH_2Cl_2$/MeOH 20:1) = 0.79. IR (ATR): $\tilde{\nu}$ = 3604.9 w, 3036.7 w, 2954.5 m, 2924.2 m, 2855.3 m, 1691.5 s, 1655.3 s, 1593.0 m, 1577.8 s, 1508.3 w, 1484.6 w, 1436.1 m, 1403.7 s, 1351.8 s, 1332.5 s, 1300.6 m, 1249.1 s, 1251.8 w, 1169.9 m, 1127.1 w, 1104.9 w, 1082.7 w, 1020.7 w, 983.9 w, 924.9 w, 854.54 w, 810.2 s, 796.1 w, 771.8 m, 748.3 s, 696.2 s $cm^{-1}$. Hauptdiastereomer: $^1$H-NMR (600 MHz, $CDCl_3$, 25°C): $\delta$ = 0.82 (t, 3 H, $^3J$=7.0 Hz, $CH_3$), 1.19-1.38 (m, 16 H, $CH_2$), 1.85-1.91 (m, 2 H, $\beta$-$CH_2$), 2.22-2.29 (m, 2 H, $\beta$-$CH_2$), 2.43 (ddd, 1 H, $^3J$ = 7.9 Hz, $^3J$ = 10.3 Hz, $^2J$ = 12.2 Hz, $CH_2$), 3.14 (ddd, 1 H, $^3J$ = 5.7 Hz, $^3J$ = 7.9 Hz, $^2J$ = 12.2 Hz, $CH_2$), 4.88 (t, 1 H, $^3J$ = 7.9 Hz, CH), 5.14 (dd, 1 H, $^3J$ = 5.7 Hz, $^3J$ = 10.3 Hz, CH), 5.16-5.21 (m, 1 H, $\alpha$-CH), 5.39 (d, 1 H, N-$CH_2$), 5.42 (d, 1 H, N-$CH_2$), 6.88-6.91 (m, 1 H, $H_{aryl}$), 7.01 (d, 2 H, $^3J$ = 1.0 Hz, $H_{aryl}$), 7.02 (d, 2 H, $^3J$ = 1.0 Hz, $H_{aryl}$), 7.21 (d, 2 H, $^3J$ =*7.4* Hz, $H_{aryl}$), 7.23 (d, 2 H, $^3J$ =7.4 Hz, $H_{aryl}$), 7.28-7.30 (m, 1 H, $H_{aryl}$), 7.32-7.35 (m, 2 H, $H_{aryl}$), 7.39-7.41 (m, 2 H, $H_{aryl}$), 7.52 (d, 2 H, $^3J$ =8.4 Hz, $H_{aryl}$), 7.61 (d, 2 H, $^3J$ =8.4 Hz, $H_{aryl}$), 8.52-8.65 ppm (m, 8 H, $H_{perylen}$). Nebendiastereomer: $^1$H-NMR (600 MHz, $CDCl_3$, 25°C): $\delta$ = 0.82 (t, 3 H, $^3J$=7.0 Hz, $CH_3$), 1.19-1.38 (m, 16 H, $CH_2$), 1.85-1.91 (m, 2 H, $\beta$-$CH_2$), 2.22-2.29 (m, 2 H, $\beta$-$CH_2$), 2.61 (ddd, 1 H, $^3J$ = 4.5 Hz, $^3J$ = 6.5 Hz, $^2J$ = 12.0 Hz, $CH_2$), 2.74 (td, 1 H, $^3J$= 9.1 Hz, $^2J$ = 12.0 Hz, $CH_2$), 4.66 (dd, 1 H, $^3J$ = 4.5 Hz, $^3J$ = 9.1 Hz, CH), 5.16-5.21 (m, 1 H, $\alpha$-CH), 5.31 (dd, 1 H, $^3J$ = 6.5 Hz, $^3J$ = 9.1 Hz, CH), 5.39 (d, 1 H, N-$CH_2$), 5.42 (d, 1 H, N-$CH_2$), 6.88-6.91 (m, 1 H, $H_{aryl}$), 6.96 (d, 2 H, $^3J$ = 1.0 Hz, $H_{aryl}$), 6.97 (d, 2 H, $^3J$ = 1.0 Hz, $H_{aryl}$), 7.15 (d, 2 H, $^3J$ =7.4 Hz, $H_{aryl}$), 7.17 (d, 2 H, $^3J$ =7.4 Hz, $H_{aryl}$), 7.28-7.30 (m, 1 H, $H_{aryl}$), 7.32-7.35 (m, 2 H, $H_{aryl}$), 7.39-7.41 (m, 2 H, $H_{aryl}$), 7.52 (d, 2 H, $^3J$=8.4 Hz, $H_{aryl}$), 7.61 (d, 2 H, $^3J$ =8.4 Hz, $H_{aryl}$), 8.52-8.65 ppm (m, 8 H, $H_{perylen}$). $^{13}$C-NMR (150 MHz, $CDCl_3$, 25°C): $\delta$ = 14.0, 22.6, 26.9, 29.2, 31.8, 32.4, 43.4, 47.2, 48.8, 54.8, 69.4, 71.4, 80.5, 113.9 ,115.8 ,122.9, 123.2, 126.5, 126.8, 128.5, 128.9, 129.6, 129.7, 131.0, 131.8, 134.2, 134.8, 136.2, 137.7, 142.4, 152.5, 163.4 ppm. UV/Vis ($CHCl_3$): $\lambda_{max}$ ($\varepsilon$) = 459 (18800), 491 (52100), 527 nm (85700). HRMS ($C_{59}H_{56}N_3O_5$): Ber. *m*/*z*: 886.426, Gef. *m*/*z:* 886.430, $\Delta$ = 4 mmu. $C_{59}H_{55}N_3O_5$ • $H_2O$ (885.4): Ber. C 78.38, H 6.35, N 4.65; Gef. C 78.17, H 6.70, N 4.34.

***N*-(1-Hexylheptyl)-*N'*-4-(5-cyano-2-phenylisoxazolidin-3-yl)benzylperylen-3,4:9,10-tetracarbonsäure-bisimid (8) und *N*-(1-Hexylheptyl)-*N'*-4-(4-cyano-2-phenyl-isoxazolidin-3-yl)benzylperylen-3,4:9,10-tetracarbonsäurebisimid (9):** *N*-(1-Hexylheptyl)-N-(4-N"-phenylcarbaldimin-*N*"-oxidobenzyl)perylen-3,4:9,10-tetracarbonsäure-bisimid (**4**, 110 mg, 153 $\mu$mol) wurde in Acrylnitril (50 mL) suspendiert, 1 h unter Rückfluss gekocht, abgekühlt, im Vakuum eingedampft und chromatographiert (Kieselgel, $CH_2Cl_2$/MeOH 45:1, Mischung aus zwei Diastereomeren, *de* = 52%). Ausb. 94 mg (73%). Schmp. > 300°C. $R_f$ (Kieselgel, $CH_2Cl_2$/MeOH 20:1) = 0.79. Hauptdiastereomer: $^1$H-NMR (600 MHz, $CDCl_3$, 25°C): $\delta$ = 0.82 (t, 3 H, $^3J$=7.0 Hz, $CH_3$), 1.19-1.38 (m, 16 H, $CH_2$), 1.85-1.91 (m, 2 H, $\beta$-$CH_2$), 2.22-2.29 (m, 2 H, $\beta$-$CH_2$), 2.47 (ddd, 1 H, $^3J$ = 3.9 Hz, $^3J$ = 5.9 Hz, $^2J$ = 12.8 Hz, $CH_2$), 3.15 (ddd, 1 H, $^3J$ = 3.9 Hz, $^3J$ = 9.0 Hz, $^2J$ = 12.8 Hz, $CH_2$), 4.47 (dd, 1 H, $^3J$ = 5.9 Hz, $^3J$ = 9.0 Hz, CH), 4.95 (dd, 1 H, $^3J$ = 3.9 Hz, $^3J$ = 9.0 Hz, CH), 5.16-5.21 (m, 1 H, $\alpha$-CH), 5.41 (s, 2 H, N-$CH_2$), 6.94-6.95 (m, 3 H, $H_{aryl}$), 7.18-7.24 (m, 2 H, $H_{aryl}$), 7.49 (d, 2 H, $^3J$ =8.3 Hz, $H_{aryl}$),

7.61 (d, 2 H, $^3J$ =8.3 Hz, $H_{aryl}$), 8.55-8.66 ppm (m, 8 H, $H_{perylen}$). Nebendiastereomer: $^1$H-NMR (600 MHz, $CDCl_3$, 25°C): $\delta$ = 0.82 (t, 3 H, $^3J$=7.0 Hz, $CH_3$), 1.19-1.38 (m, 16 H, $CH_2$), 1.85-1.91 (m, 2 H, $\beta$-$CH_2$), 2.22-2.29 (m, 2 H, $\beta$-$CH_2$), 2.77 (ddd, 1 H, $^3J$ = 6.1 Hz, $^3J$ = 7.6 Hz, $^2J$ = 12.5 Hz, $CH_2$), 2.98 (ddd, 1 H, $^3J$ = 5.2 Hz, $^3J$ = 7.6 Hz, $^2J$ = 12.5 Hz, $CH_2$), 4.87 (dd, 1 H, $^3J$ = 5.2 Hz, $^3J$ = 7.6 Hz, CH), 4.95-4.97 (m, 1 H, CH), 5.16-5.21 (m, 1 H, $\alpha$-CH), 5.41 (s, 2 H, N-$CH_2$), 6.94-6.95 (m, 3 H, $H_{aryl}$), 7.18-7.24 (m, 2 H, $H_{aryl}$), 7.49 (d, 2 H, $^3J$ =8.3 Hz, $H_{aryl}$), 7.61 (d, 2 H, $^3J$ =8.3 Hz, $H_{aryl}$), 8.55-8.66 ppm (m, 8 H, $H_{perylen}$). $C_{54}H_{50}N_4O_5$ (834.4): Ber. C 77.67, H 6.04, N 6.71; Gef. C 77.30, H 5.89, N 6.31.

***N*-(1-Hexylheptyl)-*N'*-4-(4-methyloxycarbonyl-5-methyl-2-phenylisoxazolidin-3-yl)benzylperylen-3,4: 9,10-tetracarbonsäurebisimid (7):** *N*-(1-Hexylheptyl)-*N'*-(4-*N''*-phenylcarbaldimin-*N''*-oxidobenzyl)perylen-3,4:9,10-tetracarbonsäurebisimid (4, 120 mg, 0.15 mmol) wurde in Crotonsäuremethylester (20 mL) suspendiert, 6 h auf 65 °C erhitzt (Rückfluss), im Vakuum eingedampft, chromatographiert (Kieselgel, Dichlormethan/Methanol 50:1, nicht fluoreszierende Bande), in wenig Chloroform gelöst und mit Methanol gefällt. Ausb. 80 mg (62.6%), hellroter Feststoff, Schmp. > 300°C. $R_f$ (Kieselgel, Dichlormethan/Methanol 30:1) = 0.82. IR (ATR): $\tilde{\nu}$ = 2935.4 w, 2923.6 m, 2853.9 m, 1736.0 w, 1693.4 s, 1654 s, 1592.7 s, 1577.5 m, 1507.3 w, 1486.1 w, 1434.1 m, 1403.2 m, 1376.9 w, 1331.6 s, 1247.3 m, 1216.2 w, 1169.3 w, 1124.5 w, 1105.0 w, 1020.8 w, 987.5 w, 851.8 w, 808.8 m, 744.0 m, 694.4 w $cm^{-1}$. $^1$H-NMR (600 MHz, $CDCl_3$, 25°C): $\delta$ = 0.82 (t, $^3$J(H,H) = 7.0 Hz, 6 H, 2 x $CH_3$), 1.23-1.30 (m, 16 H, 8 x $CH_2$), 1.46 (d, $^3$J(H,H) = 5.9 Hz, 3 H, $CH_3$), 1.81-1.88 (m, 2 H, $\beta$-$CH_2$), 2.21-2.27 (m, 2 H, $\beta$-$CH_2$), 3.12 (dd, $^3J$(H,H) = 7.1 Hz, $^3J$(H,H) = 9.2 Hz, 1 H, CH), 4.38 (qd, $^3J$(H,H) = 5.9 Hz, $^3J$(H,H) = 9.2 Hz, 1 H, CH), 5.10 (d, $^3J$(H,H) = 7.1 Hz, 1 H, CH), 5.15-5.22 (m, 1 H, $\alpha$-$CH_2$), 7.19-7.22 (m, 5 H, $CH_{Aromat}$), 7.53 (dd, $^3J$(H,H) = 6.2 Hz, 4 H, $CH_{Aromat}$), 8.63-8.73 ppm (m, 8 H, $CH_{Perylen}$). $^{13}$C-NMR (151 MHz, $CDCl_3$, 25°C): $\delta$ = 14.3, 22.8, 27.1, 29.4, 29.9, 32.0, 32.6, 55.0, 122.4, 123.8, 126.7, 129.1, 129.8, 131.9, 135.2, 136.7, 163.7 ppm. UV/Vis ($CHCl_3$): $\lambda_{max}$ (e) = 459 (21500), 490 (52400), 527 nm (84700). HRMS ($C_{56}H_{56}N_3O_5$) Ber. *m*/*z*: 882.408; Gef. *m*/*z*: 882.409. $\Delta$ = 1 mmu. $C_{56}H_{55}N_3O_5$ (882.1): Ber. C 76.25, H 6.28, N 4.76; Gef. C 75.84, H 6.16, N 4.58.

***N*-(1-Hexylheptyl)-*N'*-4-(5-phenyl-2-phenylisoxazolidin-3-yl)biphenylmethyl-perylen-3,4:9,10-tetracarbonsäurebisimid (12):** *N*-(1-Hexylheptyl)-*N'*-(4-*N''*-methylcarbaldimin-*N''*-oxidobiphenyl)perylen-3,4:9,10-tetracarbonsäurebisimid (**10**, 100 mg, 0.130 mmol) wurde in 5 mL Styrol gelöst und mit N-Phenylhydroxylamin (142 mg, 1.30 mmol) versetzt, 25 h bei 85°C gerührt, 40 h bei Raumtemperatur gerührt, im Vakuum eingedampft, in Dichlormethan gelöst, auf eine Chromatographiesäule aufgetragen, chromatographiert (Kieselgel, Dichlormethan/Methanol 70:1, erste nicht-fluoreszierende Bande), aus wenig Dichlormethan mit Methanol gefällt, abfiltriert und bei 110°C getrocknet (Zwei Diastereomere, *de* = 28 %). Ausb. 34 mg (27 %) roter Feststoff, Schmp. > 300°C. $R_f$ (Kieselgel, Chloroform/Ethanol = 60: 1) = 0.28. IR (ATR): $\tilde{\nu}$ = 3066.9 w, 2952.0 m, 2923.4 m, 2854.1 m, 1691.8 s, 1646.1 s, 1592.0 s, 1575.4 m, 1483.0 w, 1457.9 w, 1435.6 w, 1402.6 m, 1334.8 s, 1249.0 m, 1170.8 m, 1125.9 w, 1108.1 w, 1069.6 w, 1022.9 w, 987.3 w, 892.3w, 850.5 w, 832.3 w, 809.6 m, 763.0 w, 684.9 w, 660.6 w, 622.5 w $cm^{-1}$. Hauptdiastereomer: $^1$H-NMR (600 Hz, $CDCl_3$, 25 °C): $\delta$ = 0.82 (t, $^3J_{H,H}$ = 7.0 Hz, 6 H, $CH_3$), 1.21-1.36 (m, 16 H, $CH_2$), 1.84-1.90 (m, 2 H, $\beta$-$CH_2$), 2.20-2.28 (m, 2 H, $\beta$-$CH_2$), 2.50 (ddd, $^3J_{H,H}$ = 7.7 Hz, $^3J_{H,H}$ = 10.2 Hz, $^2J_{H,H}$ = 12.2 Hz, 1 H, $CH_2$), 3.20 (ddd, $^3J_{H,H}$ = 5.8 Hz, $^3J_{H,H}$ = 8.0 Hz, $^2J_{H,H}$ = 12.2 Hz, 1 H, $CH_2$), 4.95 (dd, $^3J_{H,H}$ = 7.8 Hz, 1 H, CH), 5.16-5.21 (m, 2 H, $\alpha$-CH, CH), 5.46 (s, 2 H, $CH_2$-N), 6.93-7.72 (m, 18 H, $H_{arom}$), 8.63-8.73 ppm (m, 8 H, $H_{perylen}$). Nebendiastereomer (36 %): H-NMR (600 Hz, $CDCl_3$, 25°C): $\delta$ = 0.82 (t, $^3J_{H,H}$ = 7.0 Hz, 6 H, $CH_3$), 1.21-1.36 (m, 16 , $CH_2$), 1.84-1.90 (m, 2 , $\beta$-$CH_2$), 2.20-2.28 (m, 2 , $\beta$-$CH_2$), 2.69 (ddd, $^3J_{H,H}$ = 4.6 Hz, $^3J_{H,H}$ = 6.6 Hz, $^2J_{H,H}$ = 12.0, 1 H, CH), 2.78-2.83 (m, 1 H, CH), 4.72 (dd, $^3J_{H,H}$ = 4.6 Hz, $^3J_{H,H}$ = 6.6 Hz, 1 H, CH), 5.16-5.21 (m, 1 H, $\alpha$-CH), 5.34-5.39 (m, 1 H, CH), 5.46 (s, 2 H, $CH_2$-N), 6.93-7.72 (m, 18 H, $H_{arom}$), 8.63-8.73 ppm (m, 8 H, $H_{perylen}$). $^{13}$C-NMR (150 MHz, $CDCl_3$, 25°C): $\delta$ = 14.0, 22.6, 26.9, 29.2, 29.7, 31.8, 32.4, 43.5, 48.7, 54.8, 71.5, 80.8, 114.0, 121.4, 123.0, 126.7, 126.9, 127.2, 127.5, 128.6, 129.0, 129.5, 131.8, 135.0, 136.1, 137.8, 139.9, 140.2, 142.0, 152.5, 163.5 ppm. UV/Vis ($CHCl_3$): $\lambda_{max}$ ($E_{rel}$) = 459 (0.21), 490 (0.60), 527 nm (1.0). HRMS ($C_{65}H_{60}N_3O_5$): Ber. *m/z:* 962.456; Gef. *m/z:* 962.459, $\Delta$ = 3 mmu.

**4-(1,3-Dioxolan-2-yl)-4-methylbenzonitril** [ vgl. W. Korytnyk, N. Angelino, C. Dave, L. Caballas, *J. Med. Chem.* **1978**, *21*,507-513]: 4-Cyanoacetophenon (5.0 g, 34.4 mmol) wurde in Toluol (50 mL) gelöst, tropfenweise mit Ethylenglykol (3.4 g, 55.1 mmol) und $BF_3$-Etherat (0.5 mL) versetzt, am Wasserabscheider 12 h unter Rückfluss erhitzt, auf Raumtemperatur abkühlen lassen (gelbe Reaktionslösung), mit 5 proz. Natriumhydrogencarbonat-Lösung (40 mL) versetzt, mit Diethylether extrahiert, mit gesättigter Natriumchlorid-Lösung (30 mL) gewaschen, über $MgSO_4$ getrocknet, filtriert, im Vakuum eingedampft und aus Diethylether/*n*-Pentan (1:1) umkristallisiert. Ausb. 2.2 g (34%) farbloser Feststoff, Schmp. 70°C. $^1$H-NMR (200 MHz, $CDCl_3$, 25°C): $\delta$ = 1.62 (s, 3 H, $CH_3$), 3.72-3.75 (m, 2 H, $CH_2$), 4.02-4.08 (m, 2 H, $CH_2$), 7.57-7.64 ppm (m, 4 H, $H_{aryl}$). HRMS ($C_{11}H_{12}NO_2$): Ber. *m/z:* 190.086, Gef. *m/z*: 190.087, $\Delta$ = 1 mmu.

**4-(1,3-Dioxolan-2-yl)-4-methylbenzylamin** [ vgl. W. Korytnyk, N. Angelino, C. Dave, L. Caballas, *J. Med. Chem.* **1978**, *21*,507-513]: Unter Argon wurde bei 0°C zu einer Suspension von Lithiumaluminiumhydrid (800 mg, 21.1 mmol) in Diethylether (20 mL) über 15 Minuten 4-(1,3-Dioxolan-2-yl)-4-methylbenzonitril (2.00 g, 10.6 mmol) in Diethylether (10 mL) vorsichtig getropft, 2 h bei 0°C gerührt, 16 h bei Raumtemperatur gerührt, vorsichtig mit wässriger NaOH - Lösung (2 N, 20 mL) tropfenweise versetzt, mit Ether extrahiert (3 x 50 mL), über $MgSO_4$ getrocknet, filtriert und im

Vakuum eingedampft. Ausb. 1.32 g (64 %) farblose Flüssigkeit, $n_D^{20}$ = 1.552. $^1$H-NMR (200 MHz, $CDCl_3$, 25°C): δ = 1.63 (s, 3 H, $CH_3$), 3.72-3.75 (m, 2 H, $CH_2$), 3.84 (s, 2 H, N-$CH_2$) 4.02-4.08 (m, 2 H, $CH_2$), 7.26 (d, 2 H, $^3J$ = 8.3 Hz, $H_{aryl}$) 7.43 ppm (d, 2 H, $^3J$ = 8.3 Hz, $H_{aryl}$). MS ($DEI^+$/70 eV): *m*/*z* (%): 193 (5) [$M^+$], 178 (100), 134 (39), 87 (20), 43 (11).

***N*-[4-(2-Methyl[1,3]dioxolan-2-yl)benzyl]-*N'*-(1-hexylheptyl)perylen-3,4:9,10-tetracarbonsäurebisimid:** *N*-1-Hexylheptylperylen-1,3:9,10-tretracarbonsäure-3,4-anhydrid-9,10-carbonsäureimid (270 mg, 0.47 mmol) wurde in Imidazol (5 g) vorgelegt, auf 140°C erhitzt, mit 4-(1,3-Dioxolan-2-yl)-4-methylbenzylamin (110 mg, 0.56 mmol) versetzt, 3 h bei 140°C gerührt, noch warm mit ein paar Millilitern Ethanol und 2 N Salzsäure (50 mL) versetzt, nach dem vollständigen Abkühlen abfiltriert, mit wässriger 2 N HCl gewaschen, 16 h im Trockenschrank bei 110°C getrocknet, in wenig Chloroform gelöst und chromatographiert (Kieselgel $CHCl_3$/EtOH 30:1). Ausb. 246 mg (70%) roter Farbstoff, Schmp. >300°C. $R_f$ (Kieselgel, Chloroform/Ethanol 30:1) = 0.17. IR (ATR): $\tilde{\nu}$ = 2955.0 m, 2923.9 m, 2856.0 m, 1693.1 m, 1648.4 s, 1592.9 m, 1576.5 m, 1507.5 w, 1482.9 w, 1456.8 w, 1435.9 m, 1420.5 w, 1403.7 m, 1378.5 w, 1339.3 m, 1284.2 m, 1249.1 m, 1173.2 m, 1135.5 w, 1127.9 w, 1111.1 w, 1091.7 w, 1038.2 m, 1020.2 w, 983.8 w, 948.3 w, 862.4 w, 808.9 m, 781.1 w, 744.4 m, 725.4 w $cm^{-1}$. $^1$H-NMR (600 MHz, $CDCl_3$, 25°C): δ = 8.43-8.57 (m, 8 H, $H_{pery}$), 7.54 (d, 1H, $^3J$=8.5 Hz), 7.44 (d, 1 H, $^3J$=8.5 Hz), 5.37 (s, 2 H, $CH_2$-N), 5.16-5.21 (m, 1 H, CH-N), 3.72-3.76 (m, 2 H, $CH_2$), 3.98-4.00 (m, 2 H, $CH_2$), 2.23-2.29 (m, 2 H, β-$CH_2$), 1.86-1.92 (m, 2 H, β-$CH_2$), 1.21-1.38 (m, 16 H, $CH_2$), 0.82 ppm (t, 1 H, $^3J$= 7.0 Hz). $^{13}$C-NMR (150 MHz, $CDCl_3$, 25°C): δ = 163.2, 142.7, 136.6, 134.7, 131.7, 131.6, 129.4, 129.3, 129.1, 129.0, 128.6, 125.5, 123.1, 22.9, 122.9, 108.7, 64.4, 54.8, 43.4, 32.4, 31.8, 29.2, 27.6, 27.0, 22.6, 14.0 ppm. UV/Vis ($CHCl_3$): $\lambda_{max}$ (ε) = 463 (81220), 492 (50320), 529 nm (18510). Fluoreszenz ($CHCl_3$): $\lambda_{max}$ ($I_{rel}$) = 539 (1.00), 582 nm (0.40). Fluoreszenzquantenausbeute ($CHCl_3$, $\lambda_{exc}$ = 491 nm, $E_{491\ nm}$ = 0.0347 $cm^{-1}$, Referenz: **1a** mit Φ = 1.00): 1.00. MS ($DEI^+$ / 70 eV): *mlz* (%) = 749 (21) [$M^+$ + H], 748 [$M^+$] (38), 735 (13), 734 (47), 733 (100), 569 (12), 568 (18), 567 (21), 554 (11), 553 (34), 552 (41), 551 (43), 549 (13), 548 (31), 373 (11), 276 (12), 275 (30). $C_{48}H_{48}N_2O_6$ (748.9): Ber. C 76.98, H 6.46, N 3.74; Gef. C 77.05, H 6.41, N 3.58.

***N*-(4-Acetylbenzyl)-*N'*-(1-hexylheptyl)perylen-3,4:9,10-tetracarbonsäurebisimid:** Für die Acetal-Spaltung wurde *N*-[4-(2-Methyl[1,3]dioxolan-2-yl)benzyl]-*N'*-(1-hexylheptyl)perylen-3,4:9,10-tetracarbonsäurebisimid (1.72 g, 2.30 mmol) in Tetrahydofuran (220 mL) bei 70 °C gelöst, mit wässriger 2 N Salzsäure (50 mL) versetzt, 5 h auf 70°C erhitzt, durch Zugabe von 2 N Salzsäure ausgefällt, abfiltriert, nachgewaschen und bei 110°C getrocknet. Ausb. 1.60 g (98 %) roter Farbstoff, Schmp. > 300 °C. $R_f$ (Kieselgel, Chloroform/Ethanol 30:1) = 0.83. IR (ATR): $\tilde{\nu}$ = 2957.0 m, 2922.1 m, 2855.1 m, 1696.8 m, 1674.8 m, 1647.9 s, 1608.3 w, 1592.9 m, 1576.1 m, 1507.7 w, 1482.9 w, 1466.3 w, 1436.6 m, 1403.7 m, 1379.4 w, 1336.4 s, 1310.1 m, 1251.0 m, 1192.4 w, 1171.6 m, 1124.9 w, 1114.6 w, 1076.7 w, 1019.5 w, 988.5 m, 956.4 w, 852.2 w, 839.8 m, 808.9 s, 796.4 m, 783.5 m, 745.6 s, 727.9 w, 683.9 w $cm^{-1}$. $^1$H-NMR (600 Hz, $CDCl_3$, 25°C): δ = 0.82 (t, 6 H, $^3J_{H,H}$ = 7.1 Hz, $CH_3$), 1.19-1.39 (m, 16 H, $CH_2$), 1.85-1.91 (m, 2 H, β-$CH_2$), 2.22-2.28 (m, 2 H, β-$CH_2$), 5.18 (tt, $^3J_{H,H}$ = 5.8 $H_z$, $^3J_{H,H}$ = 9.3 Hz, 1 H, α-CH), 5.44 (s, 2 H, $CH_2$-N), 7.77 (d, $^3J_{H,H}$ = 8.5 Hz, $^4J_{H,H}$ = 173.9 Hz, 4 H, $H_{arom}$), 8.55-8.65 ppm (m, 8 H, $H_{perylen}$). $^{13}$C-NMR (150 MHz, $CDCl_3$, 25°C): δ = 14.0, 22.6, 26.6, 27.0, 29.2, 29.7, 31.8, 32.4, 43.5, 54.9, 122.8, 123.0, 123.3, 126.3, 126.5, 128.6, 129.0, 129.5, 131.7, 134.1, 135.0, 136.4, 142.7, 163.3 ppm. UV/Vis ($CHCl_3$): $\lambda_{max}$ (ε) = 462 (20570), 491 (51780), 527 nm (84530). Fluoreszenz ($CHCl_3$): $\lambda_{max}$ ($I_{rel}$) = 539 (1.00), 582 nm (0.40). Fluoreszenzquantenausbeute ($CHCl_3$, $\lambda_{exc}$ = 491 nm, $E_{491}$ nm = 0.0294 $cm^{-1}$, Referenz: **1a** mit Φ = 1.00): 1.00. MS ($DEI^+$ / 70 eV): *m*/*z* (%) = 705 [$M^+$+H] (24), 704 [$M^+$] (37), 524 (24), 523 (76), 522 (100), 508 (11), 507 (24), 390 [$M^+$-$C_{13}H_{27}$-$C_9H_9O$] (7), 374 (15), 373 (9), 346 (12), 254 (11), 55 (8). HRMS ($C_{46}H_{44}N_2O_5$): Ber. *m*/*z*: 704.327, Gef. *m*/*z*: 704.330, Δ = 3 mmu. $C_{46}H_{44}N_2O_5$ (704.3): Ber. C 78.38, H 6.29, N 3.97; Gef. C 77.96, H 6.21, N 3.91.

## Patentansprüche

1. Perylenbisimid-Dyaden der allgemeinen Formel (**I**),

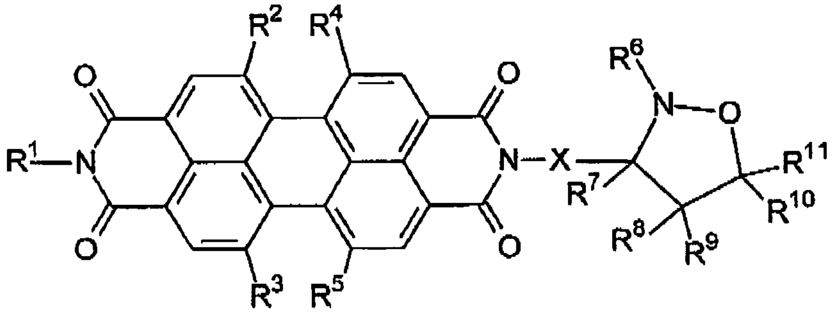

(I)

wobei die Reste $R^1$ bis $R^{11}$ gleich oder verschieden sind und unabhängig voneinander für Wasserstoff oder einen linearen Alkylrest mit mindestens einem und höchstens 37 C-Atomen stehen;
wobei in dem Alkylrest eine bis 10 $CH_2$-Einheiten unabhängig voneinander ersetzt sein können durch eine Carbonylgruppe, ein Sauerstoffatom, Schwefelatom, Selenatom, Telluratom, eine *cis*- oder *trans*-CH=CH-Gruppe, bei der eine CH-Einheit auch durch ein Stickstoffatom ersetzt sein kann, eine acetylenische C≡C-Gruppe, einen divalenten Phenylrest, divalenten Pyridinrest, divalenten Thiophenrest, divalenten Naphthalinrest bei dem eine oder zwei CH-Gruppen durch Stickstoffatome ersetzt sein können, und einen divalenten Anthracenrest bei dem eine oder zwei CH-Gruppen durch Stickstoffatome ersetzt sein können;
wobei bis zu 12 einzelne Wasserstoffatome der $CH_2$-Gruppen jeweils unabhängig voneinander auch an gleichen C-Atomcn ersetzt sein können durch die Halogene Fluor, Chlor, Brom oder Iod, eine Cyanogruppe oder eine lineare Alkylkette mit bis zu 18 C-Atomen, bei der eine bis 6 $CH_2$-Einheiten unabhängig voneinander ersetzt sein können durch eine Carbonylgruppe, ein Sauerstoffatom, Schwefelatom, Selenatom, Telluratom, eine *cis*- oder *trans*-CH=CH-Gruppe, bei der eine CH-Einheit durch ein Stickstoffatom ersetzt sein kann, eine acetylenische C≡C-Gruppe, einen divalenten Phenylrest, einen divalenten Pyridinrest, einen divalenten Thiophenrest, einen divalenten Naphthalinrest bei dem eine oder zwei CH-Gruppen durch Stickstoffatome ersetzt sein können, und einen divalenten Anthracenrest bei denen eine oder zwei CH-Gruppen durch Stickstoffatome ersetzt sein können;
wobei bis zu 12 einzelne Wasserstoffatome der $CH_2$-Gruppen in einem Alkylrest jeweils unabhängig voneinander auch an gleichen C-Atomen ersetzt sein können durch die Halogene Fluor, Chlor, Brom oder Iod, eine Cyanogruppe oder eine lineare Alkylkette mit bis zu 18 C-Atomen, bei der eine bis 6 $CH_2$-Einbeiten unabhängig voneinander ersetzt sein können durch eine Carbonylgruppe, ein Sauerstoffatom, Schwefelatom, Selenatom, Telluratom, eine *cis*- oder *trans*-CH=CH-Gruppe, bei der eine CH-Einheit durch ein Stickstoffatom ersetzt sein kann, eine acetylenische C≡C-Gruppe, einen divalenten Phenylrest, einen divalenten Pyridinrest, einen divalente Thiophenrest, einen divalenten Naphthalinrest bei dem ein oder zwei CH-Gruppen durch Stickstoffatome ersetzt sein können, und einen divalenten Anthraeenrest bei dem eine oder zwei CH-Gruppen durch Stickstoffatome ersetzt sein können;
wobei $CH_2$-Gruppen, an denen wie vorstehend beschrieben ein Wasserstoffatom ersetzt ist, auch unter Bildung eines Rings miteinander verknüpft sein können;
wobei die Reste $R^1$ bis $R^5$ und $R^7$ bis $R^{11}$ außerdem unabhängig voneinander die Halogenatome F, Cl, Br oder I oder CN bedeuten können;
wobei X in Formel (I) eine bis 12 $CH_2$-Einheiten bedeutet, bei denen unabhängig voneinander eine oder mehrere ersetzt sein können durch jeweils eine Carbonylgruppe, ein Sauerstoffatom, Schwefelatom, Selenatom, Telluratom, eine *cis*- oder *trans*-CH=CH-Gruppe, bei der eine CH-Einheit auch durch ein Stickstoffatom ersetzt sein kann, eine acetylenische C≡C-Gruppe, einen divalenten Phenylrest, einen divalenten Pyridinrest, einen divalenten Thiophenrest, einen divalenten Naphthalinrest bei dem eine oder zwei CH-Gruppen durch Stickstoffatome ersetzt sein können, und einen divalenten Anthracenrest bei dem eine oder zwei CH-Gruppen durch Stickstoffatome ersetzt sein können;
wobei bis zu 12 einzelne Wasserstoffatome der $CH_2$-Gruppen jeweils unabhängig voneinander auch an gleichen C-Atomen ersetzt sein können durch die Halogene Fluor, Chlor, Brom oder Iod, eine Cyanogruppe oder eine lineare Alkylkette mit bis zu 18 C-Atomen, bei der eine bis 6 $CH_2$-Einbeiten unabhängig voneinander ersetzt sein können durch eine Carbonylgruppe, ein Sauerstoffatom, Schwefelatom, Selenatom, Telluratom, eine *cis*- oder *trans*-CH=CH-Gruppe, bei der eine CH-Einheit durch ein Stickstoffatom ersetzt sein kann, eine acetylenische C≡C-Gruppe, einen divalenten Phenylrest, divalenten Pyridinrest, divalenten Thiophenrest, divalenten Naphthalinrest bei dem eine oder zwei CH-Gruppen durch Stickstoffatome ersetzt sein können, und einen divalenten Anthracenrest bei dem ein oder zwei CH-Gruppen durch Stickstoffatome ersetzt sein können;
wobei bis zu 12 einzelne Wasserstoffatome der $CH_2$-Gruppen der Alkylreste jeweils unabhängig voneinander auch

an gleichen C-Atomen ersetzt sein können durch die Halogene Fluor, Chlor, Brom oder Iod, eine Cyanogruppe oder eine lineare Alkylkette mit bis zu 18 C-Atomen, bei der eine bis 6 $CH_2$-Einheiten unabhängig voneinander ersetzt sein können durch eine Carbonylgruppe, ein Sauerstoffatom, Schwefelatom, Selenatom, Telluratom, eine *cis*- oder *trans*-CH=CH-Gruppe, bei der eine CH-Einheit durch ein Stickstoffatom ersetzt sein kinn, eine acetylenische C≡C-Gruppe, einen divalenten Phenylrest, divalenten Pyridinrest, divalenten Thiophenrest, divalenten Naphthalinrest bei dem eine oder zwei CH-Gruppen durch Stickstoffatome ersetzt sein können, und einen divalenten Anthracenrest bei dem eine oder zwei CH-Gruppen durch Stickstoffatome ersetzt sein können;
wobei es sich bei einer $CH_2$ Einheit, die ersetzt werden kann, auch um eine endständige Einheit in einem Alkylrest/ einer Alkylketten, also eine entsprechende Einheit innerhalb einer $-CH_3$ Gruppe handeln kann;
und wobei $CH_2$-Gruppen, an denen wie vorstehend beschrieben ein Wasserstoffatom ersetzt ist, auch unter Bildung eines Rings miteinander verknüpft sein können.

**2.** Perylentetracarbonsäurebisimid-Nitrone der allgemeinen Formel (**II**),

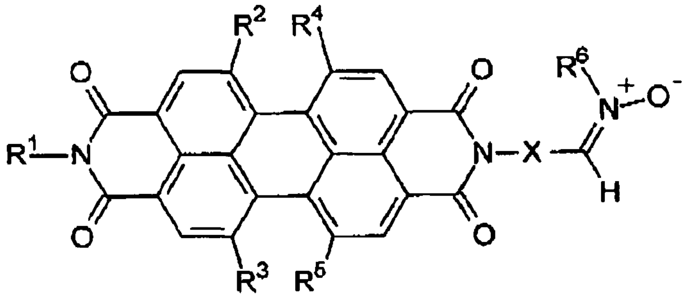

**(II)**

in der die Reste $R^1$ bis $R^6$ und X die für Formel (I) angegebene Bedeutung haben.

**3.** Verbindung nach Anspruch 1 oder 2, wobei die Reste $R^1$ bis $R^5$ ausgewählt sind aus Wasserstoff und einem Kohlenwasserstoffrest.

**4.** Verbindung nach einem der Ansprüche 1 bis 3, wobei $R^6$ ausgewählt ist aus einem Arylrest oder Heteroarylrest.

**5.** Verbindung nach einem der Ansprüche 1, bis 4, wobei X eine Gruppe ist, die aus einer oder zwei Phenylen-Gruppen oder einer Bisphenylen-Gruppe in Kombination mit 1 bis 4 Methyleneinheiten gebildet ist.

**6.** Verwendung von Isoxazolidinen der allgemeinen Formel (**IV**) als Elektronendonorgruppe in lichtgetriebenen Systemen zur Ladungstrennung,

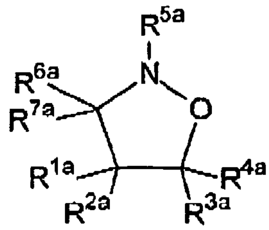

**(IV)**

wobei die Reste $R^{1a}$ bis $R^{7a}$ gleich oder verschieden sind und unabhängig voneinander für Wasserstoff oder einen linearen Alkylrest mit mindestens einem und höchstens 37 C-Atomen stehen;
wobei in dem Alkylrest eine bis 10 $CH_2$-Einheiten unabhängig voneinander ersetzt sein können durch eine Carbonylgruppe, ein Sauerstoffatom, Schwefelatom, Selenatom, Telluratom, eine *cis*- oder *trans*-CH=CH-Gruppe, bei der eine CH-Einheit auch durch ein Stickstoffatom ersetzt sein kann, eine acetylenische C≡C-Gruppe, einen divalenten

Phenylrest, divalenten Pyridinrest, divalenten Thiophenrest, divalenten Naphthalinrest bei dem eine oder zwei CH-Gruppen durch Stickstoffatome ersetzt sein können, und einen divalenten Anthracenrest bei dem eine oder zwei CH-Gruppen durch Stickstoffatome ersetzt sein können;
wobei bis zu 12 einzelne Wasserstoffatome der $CH_2$-Gruppen jeweils unabhängig voneinander auch an gleichen C-Atomen ersetzt sein können durch die Halogene Fluor, Chlor, Brom oder Iod, eine Cyanogruppe oder eine lineare Alkylkette mit bis zu 18 C-Atomen, bei der eine bis 6 $CH_2$-Einheiten unabhängig voneinander ersetzt sein können durch eine Carbonylgruppe, ein Sauerstoffatom, Schwefelatom, Selenatom, Telluratom, eine *cis-* oder *trans*-CH=CH-Gruppe, bei der eine CH-Einheit durch ein Stickstoffatom ersetzt sein kann, eine acetylenische C≡C-Gruppe, einen divalenten Phenylrest, einen divalenten Pyridinrest, einen divalenten Thiophenrest, einen divalenten Naphthalinrest bei dem eine oder zwei CH-Gruppen durch Stickstoffatome ersetzt sein können, und einen divalenten Anthracenrest bei denen eine oder zwei CH-Gruppen durch Stickstoffatome ersetzt sein können;
wobei bis zu 12 einzelne Wasserstoffatome der $CH_2$-Gruppen in einem Alkylrest jeweils unabhängig voneinander auch an gleichen C-Atomen ersetzt sein können durch die Halogene Fluor, Chlor, Brom oder Iod, eine Cyanogruppe oder eine lineare Alkylkette mit bis zu 18 C-Atomen, bei der eine bis 6 $CH_2$-Einheiten unabhängig voneinander ersetzt sein können durch eine Carbonylgruppe, ein Sauerstoffatom, Schwefelatom, Selenatom, Telluratom, eine *cis-* oder *trans*-CH=CH-Gruppe, bei der eine CH-Einheit durch ein Stickstoffatom ersetzt sein kann, eine acetylenische C≡C-Gruppe, einen divalenten Phenylrest, einen divalenten Pyridinrest, einen divalente Thiophenrest, einen divalenten Naphthalinrest bei dem ein oder zwei CH-Gruppen durch Stickstoffatome ersetzt sein können, und einen divalenten Anthracenrest bei dem eine oder zwei CH-Gruppen durch Stickstoffatome ersetzt sein können;
wobei $CH_2$-Gruppen, an denen wie vorstehend beschrieben ein Wasserstoffatom ersetzt ist, auch unter Bildung eines Rings miteinander verknüpft sein können;
wobei es sich bei einer $CH_2$ Einheit, die ersetzt werden kann, auch um eine endständige Einheit in einem Alkylrest/ einer Alkylkette, also eine entsprechende Einheit innerhalb einer $-CH_3$ Gruppe handeln kann;
und wobei die Reste $R^{1a}$ bis $R^{4a}$, $R^{6a}$ und $R^{7a}$ außerdem unabhängig voneinander die Halogenatome F, Cl, Br oder I oder CN bedeuten können.

7. Verfahren zur Herstellung einer Perylenbisimid-Dyade der Formel (I) nach einem der Ansprüche 1 oder 3 bis 5, wobei ein Olefin durch 1,3-dipolare Cycloaddition mit einem Nitron der Formel (II) nach Anspruch 2 umgesetzt wird.

8. Verfahren zur Herstellung eines Perylenbisimid-Nitrons der Formel (II) nach einem der Ansprüche 2 bis 5, wobei ein Perylenbisimid-Aldehyd mit einem Hydroxylaminderivaten umgesetzt wird.

9. Photovoltaische Zelle, umfassend eine Verbindung nach einem der Ansprüche 1 bis 5.

10. Photovoltaische Zelle nach Anspruch 9, wobei es sich um eine Titandioxid-Zelle handelt.

11. Verwendung einer Verbindung nach einem der Ansprüche 1 bis 5 als System zur lichtgetriebenen Ladungstrennung bei der Gewinnung von Solarenergie.

12. Verwendung einer Verbindung nach einem der Ansprüche 1 bis 5 als lichtgetriebenes Reduktions- oder Oxidationsmittel in chemischen Reaktionen.

13. Verwendung einer Verbindung nach einem der Ansprüche 1 bis 5 als Fluoreszenzindikator für Protonensäuren oder Lewis-Säuren.

**Claims**

1. Perylene bisimide dyads of the general formula (I)

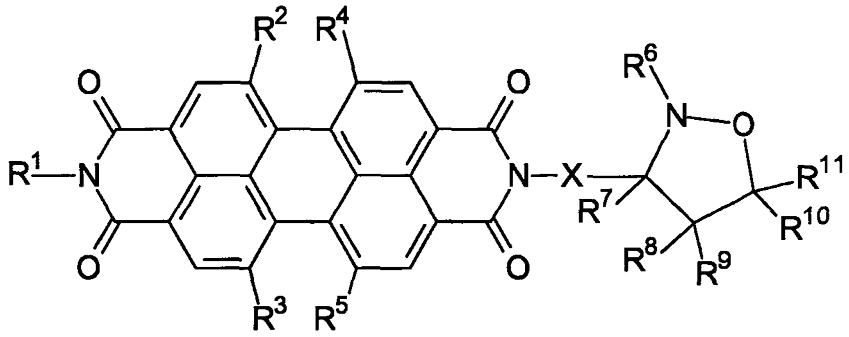

**(I)**

wherein $R^1$ to $R^{11}$ are identical or different and represent, independently from each other, hydrogen or a linear alkyl group with at least one and at the most 37 C-atoms;
wherein in the alkyl group, one to 10 $CH_2$ units can be replaced, independently from each other, by a carbonyl group, an oxygen atom, a sulphur atom, a selenium atom, a tellurium atom, a *cis*- or *trans*-CH=CH group in which one CH unit can also be replaced by a nitrogen atom, an acetylenic C≡C group, a divalent phenyl group, a divalent pyridine group, a divalent thiophene group, divalent naphthalene group, in which one or two CH groups can be replaced by nitrogen atoms, and a divalent anthracene group, in which one or two CH groups can be replaced by nitrogen atoms;
wherein up to 12 individual hydrogen atoms of the $CH_2$ groups can be replaced, independently from each other, also at the same C atoms by the halogens fluorine, chlorine, bromine or iodine, a cyano group or a linear alkyl chain with up to 18 C atoms, in which one to 6 $CH_2$ units can be replaced, independently from each other, by a carbonyl group, an oxygen atom, a sulphur atom, a selenium atom, a tellurium atom, a *cis*- or *trans*-CH=CH group, in which a CH unit can be replaced by a nitrogen atom, an acetylenic C≡C group, a divalent phenyl group, a divalent pyridine group, a divalent thiophene group, a divalent naphthalene group in which one or two CH groups can be replaced by a nitrogen atom, and a divalent anthracene group, in which one or two CH groups can be replaced by nitrogen atoms;
wherein up to 12 individual hydrogen atoms of the $CH_2$ groups in an alkyl group can be replaced, independently from each other, also at the same C atoms by the halogens fluorine, chlorine, bromine or iodine, a cyano group or a linear alkyl chain with up to 18 C atoms, in which one to 6 $CH_2$ units can be replaced, independently from each other, by a carbonyl group, an oxygen atom, a sulphur atom, a selenium atom, a tellurium atom, a *cis*- or *trans*-CH=CH group in which a CH unit can be replaced by a nitrogen atom, an acetylenic C≡C group, a divalent phenyl group, a divalent pyridine group, a divalent thiophene group, a divalent naphthalene group in which one or two CH groups can be replaced by nitrogen atoms, and a divalent anthracene group in which one or two CH groups can be replaced by nitrogen atoms;
wherein $CH_2$ groups, in which a hydrogen atom is replaced as described above, can be linked together under the formation of a ring;
wherein $R^1$ to $R^5$ and $R^7$ to $R^{11}$ can represent, independently from each other, the halogen atoms F, Cl, Br or I or CN;
wherein X in formula (I) represents one to 12 $CH_2$ units, in which one or more can be replaced, independently from each other, by a carbonyl group, an oxygen atom, a sulphur atom, a selenium atom, a tellurium atom, a *cis*- or *trans*-CH=CH group in which a CH unit can be replaced by a nitrogen atom, an acetylenic C≡C group, a divalent phenyl group, a divalent pyridine group, a divalent thiophene group, a divalent naphthalene group in which one or two CH groups can be replaced by nitrogen atoms, and a divalent anthracene group in which one or two CH groups can be replaced by nitrogen atoms;
wherein up to 12 individual hydrogen atoms of the $CH_2$ groups can be replaced, independently from each other, also at the same C atoms by the halogens fluorine, chlorine, bromine or iodine, a cyano group or a linear alkyl chain with up to 18 C atoms in which one to 6 $CH_2$ units can be replaced, independently from each other, by a carbonyl group, an oxygen atom, a sulphur atom, a selenium atom, a tellurium atom, a *cis*- or *trans*-CH=CH group, in which a CH unit can also be replaced by a nitrogen atom, an acetylenic C≡C group, a divalent phenyl group, a divalent pyridine group, a divalent thiophene group, a divalent naphthalene group in which one or two CH groups can be replaced by nitrogen atoms, and a divalent anthracene group in which one or two CH groups can be replaced by nitrogen atoms;
wherein up to 12 individual hydrogen atoms of the $CH_2$ groups of the alkyl groups can be replaced, independently from each other, also at the same C atoms by the halogens fluorine, chlorine, bromine or iodine, a cyano group or

a linear alkyl chain with up to 18 C atoms in which one to 6 $CH_2$ units can be replaced, independently from each other, by a carbonyl group, an oxygen atom, a sulphur atom, a selenium atom, a tellurium atom, a *cis-* or *trans-* CH=CH group in which a CH unit can be replaced by a nitrogen atom, an acetylenic C≡C group, a divalent phenyl group, a divalent pyridine group, a divalent thiophene group, a divalent naphthalene group in which one or two CH groups can be replaced by nitrogen atoms, and a divalent anthracene group in which one or two CH groups can be replaced by nitrogen atoms;
wherein the $CH_2$ unit that can be replaced can be a terminal unit in an alkyl group / a alkyl chain, i.e. a corresponding unit within a $-CH_3$ group;
and wherein $CH_3$ groups, in which a hydrogen atom is replaced as described above, can also be linked together under the formation of a ring.

**2.** Perylene tetracarboxylic acid bisimide nitrons of the general formula (II)

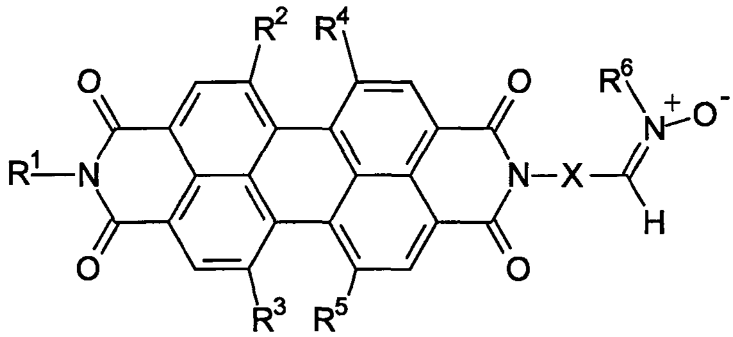

**(II)**

in which $R^1$ to $R^6$ and X have the meaning given for formula (I).

**3.** The compound according to claim 1 or 2, wherein $R^1$ to $R^5$ are chosen from hydrogen and a hydrocarbon group.

**4.** The compound according to one of claims 1 to 3, wherein $R^6$ is chosen from an aryl group or a heteroaryl group.

**5.** The compound according to one of claims 1 to 4, wherein X is a group that is made up of one or two phenylene groups or a bisphenylene group in combination with 1 to 4 methyl units.

**6.** Use of isoxazolidines of the general formula (IV) as an electron donating group in light driven systems for charge separation

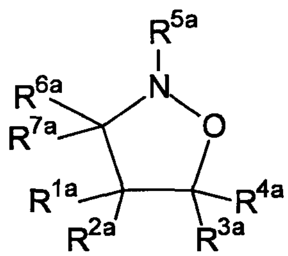

**(IV)**

wherein $R^{1a}$ to $R^{7a}$ are identical or different and represent independently from each other hydrogen or a linear alkyl group with at least one and at most 37 C atoms;
wherein in the alkyl group, one to 10 $CH_2$ units can be replaced, independently from each other, by a carbonyl group, an oxygen atom, a sulphur atom, a selenium atom, a tellurium atom, a *cis-* or *trans-*CH=CH group in which one CH

unit can also be replaced by a nitrogen atom, an acetylenic C=C group, a divalent phenyl group, a divalent pyridine group, a divalent thiophene group, divalent naphthalene group, in which one or two CH groups can be replaced by nitrogen atoms, and a divalent anthracene group, in which one or two CH groups can be replaced by nitrogen atoms;
wherein up to 12 individual hydrogen atoms of the $CH_2$ groups can be replaced, independently from each other, also at the same C atoms by the halogens fluorine, chlorine, bromine or iodine, a cyano group or a linear alkyl chain with up to 18 C atoms, in which one to 6 $CH_2$ units can be replaced, independently from each other, by a carbonyl group, an oxygen atom, a sulphur atom, a selenium atom, a tellurium atom, a *cis*- or *trans*-CH=CH group, in which a CH unit can be replaced by a nitrogen atom, an acetylenic C≡C group, a divalent phenyl group, a divalent pyridine group, a divalent thiophene group, a divalent naphthalene group in which one or two CH groups can be replaced by a nitrogen atom, and a divalent anthracene group, in which one or two CH groups can be replaced by nitrogen atoms;
wherein up to 12 individual hydrogen atoms of the $CH_2$ groups in an alkyl group can be replaced, independently from each other, also at the same C atoms by the halogens fluorine, chlorine, bromine or iodine, a cyano group or a linear alkyl chain with up to 18 C atoms, in which one to 6 $CH_2$ units can be replaced, independently from each other, by a carbonyl group, an oxygen atom, a sulphur atom, a selenium atom, a tellurium atom, a *cis*- or *trans*-CH=CH group in which a CH unit can be replaced by a nitrogen atom, an acetylenic C≡C group, a divalent phenyl group, a divalent pyridine group, a divalent thiophene group, a divalent naphthalene group in which one or two CH groups can be replaced by nitrogen atoms, and a divalent anthracene group in which one or two CH groups can be replaced by nitrogen atoms;
wherein $CH_2$ groups, in which a hydrogen atom is replaced as described above, can be linked together under the formation of a ring;
wherein the $CH_2$ unit that can be replaced can be a terminal unit in an alkyl group / a alkyl chain, i.e. a corresponding unit within a $-CH_3$ group;
and wherein $R^{1a}$ to $R^{4a}$, $R^{6a}$ and $R^{7a}$ can also represent, independently from each other, the halogen atoms F, Cl, Br or I or CN.

**7.** A method for the preparation of a perylene bisimide dyad of the formula (I) according to one of the claims 1 or 3 to 5, wherein an olefine is converted with a nitron of formula (II) according to claim 2 by 1,3-dipolar cycloaddition.

**8.** A method for the preparation of a perylene bisimide nitron of formula (II) according to one of claims 2 to 5, wherein a perylene bisimide aldehyde is converted with a hydroxylamine derivative.

**9.** A photovoltaic cell, comprising a compound according one of claims 1 to 5.

**10.** The photovoltaic cell according to claim 9, wherein the cell is a titanium dioxide cell.

**11.** Use of a compound according to one of claims 1 to 5 as a system for light driven charge separation in the generation of solar energy.

**12.** Use of a compound according to one of claims 1 to 5 as light driven reducing or oxidizing agent in chemical reactions.

**13.** Use of a compound according to one of claims 1 to 5 as a fluorescence indicator for protonic acids or Lewis acids.

## Revendications

**1.** Dyades de pérylène-bisimide de formule générale (I),

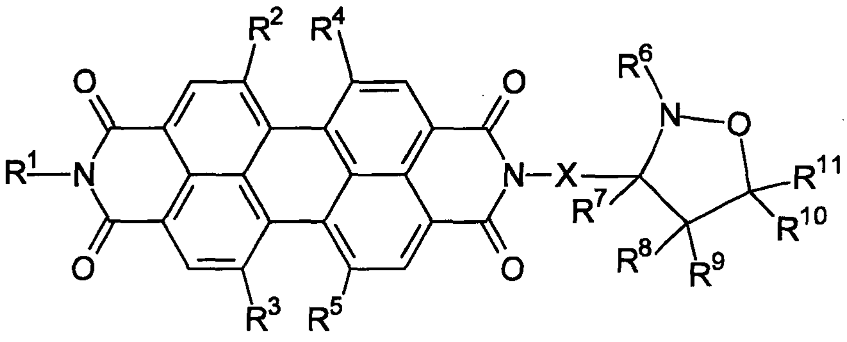

(I)

les radicaux $R^1$ à $R^{11}$ étant identiques ou différents et représentant, indépendamment les uns des autres, hydrogène ou un radical alkyle linéaire comprenant au moins un et au plus 37 atomes de carbone ;
une à 10 unités $CH_2$ pouvant être remplacées indépendamment les unes des autres dans le radical alkyle par un groupe carbonyle, un atome d'oxygène, un atome de soufre, un atome de sélénium, un atome de tellure, un groupe CH=CH cis ou trans, dans lequel une unité CH peut également être remplacée par un atome d'azote, un groupe C≡C acétylénique, un radical phényle divalent, un radical pyridine divalent, un radical thiophène divalent, un radical naphtalène divalent dans lequel un ou deux groupes CH peuvent être remplacés par des atomes d'azote et un radical anthracène divalent, dans lequel un ou deux groupes CH peuvent être remplacés par des atomes d'azote ;
jusqu'à 12 atomes d'hydrogène individuels des groupes $CH_2$ pouvant être remplacés, à chaque fois indépendamment les uns des autres, même sur des atomes de carbone identiques, par les halogènes fluor, chlore, brome ou iode, un groupe cyano ou une chaîne alkyle linéaire comprenant jusqu'à 18 atomes de carbone, dans laquelle une à 6 unités $CH_2$ peuvent être remplacées indépendamment les unes des autres par un groupe carbonyle, un atome d'oxygène, un atome de soufre, un atome de sélénium, un atome de tellure, un groupe CH=CH cis ou trans, dans lequel une unité CH peut être remplacée par un atome d'azote, un groupe C=C acétylénique, un radical phényle divalent, un radical pyridine divalent, un radical thiophène divalent, un radical naphtalène divalent dans lequel un ou deux groupes CH peuvent être remplacés par des atomes d'azote, et un radical anthracène divalent dans lequel un ou deux groupes CH peuvent être remplacés par des atomes d'azote ;
jusqu'à 12 atomes d'hydrogène individuels des groupes $CH_2$ dans un radical alkyle pouvant être remplacés, à chaque fois indépendamment les uns des autres, même sur des atomes de carbone identiques, par les halogènes fluor, chlore, brome ou iode, un groupe cyano ou une chaîne alkyle linéaire comprenant jusqu'à 18 atomes de carbone, dans laquelle une à 6 unités $CH_2$ peuvent être remplacées indépendamment les unes des autres par un groupe carbonyle, un atome d'oxygène, un atome de soufre, un atome de sélénium, un atome de tellure, un groupe CH=CH cis ou trans, dans lequel une unité CH peut être remplacée par un atome d'azote, un groupe C≡C acétylénique, un radical phényle divalent, un radical pyridine divalent, un radical thiophène divalent, un radical naphtalène divalent dans lequel un ou deux groupes CH peuvent être remplacés par des atomes d'azote, et un radical anthracène divalent dans lequel un ou deux groupes CH peuvent être remplacés par des atomes d'azote ;
les groupes $CH_2$, sur lesquels, comme décrit ci-dessus, un atome d'hydrogène est remplacé, pouvant également être liés les uns aux autres en formant un cycle ;
les radicaux $R^1$ à $R^5$ et $R^7$ à $R^{11}$ pouvant en outre signifier, indépendamment les uns des autres, les atomes d'halogène F, Cl, Br ou I ou CN ;
X dans la formule (I) signifiant une à 12 unités $CH_2$, parmi lesquelles, indépendamment les unes des autres, une ou plusieurs peuvent être remplacées par à chaque fois un groupe carbonyle, un atome d'oxygène, un atome de soufre, un atome de sélénium, un atome de tellure, un groupe CH=CH cis ou trans, dans lequel une unité CH peut également être remplacés par un atome d'azote, un groupe C≡C acétylénique, un radical phényle divalent, un radical pyridine divalent, un radical thiophène divalent, un radical naphtalène divalent dans lequel un ou deux groupes CH peuvent être remplacée par des atomes d'azote et un radical anthracène divalent, dans lequel un ou deux groupes CH peuvent être remplacés par des atomes d'azote ;
jusqu'à 12 atomes d'hydrogène individuels des groupes $CH_2$ dans un radical alkyle pouvant être remplacés, à chaque fois indépendamment les uns des autres, même sur des atomes de carbone identiques, par les halogènes fluor, chlore, brome ou iode, un groupe cyano ou une chaîne alkyle linéaire comprenant jusqu'à 18 atomes de carbone, dans laquelle une à 6 unités $CH_2$ peuvent être remplacées indépendamment les unes des autres par un groupe carbonyle, un atome d'oxygène, un atome de soufre, un atome de sélénium, un atome de tellure, un groupe

CH=CH cis ou trans, dans lequel une unité CH peut être remplacée par un atome d'azote, un groupe C≡C acétylénique, un radical phényle divalent, un radical pyridine divalent, un radical thiophène divalent, un radical naphtalène divalent dans lequel un ou deux groupes CH peuvent être remplacés par des atomes d'azote, et un radical anthracène divalent dans lequel un ou deux groupes CH peuvent être remplacés par des atomes d'azote ;
jusqu'à 12 atomes d'hydrogène individuels des groupes $CH_2$ des radicaux alkyle pouvant être remplacés, à chaque fois indépendamment les uns des autres, même sur des atomes de carbone identiques, par les halogènes fluor, chlore, brome ou iode, un groupe cyano ou une chaîne alkyle linéaire comprenant jusqu'à 18 atomes de carbone, dans laquelle une à 6 unités $CH_2$ peuvent être remplacées indépendamment les unes des autres par un groupe carbonyle, un atome d'oxygène, un atome de soufre, un atome de sélénium, un atome de tellure, un groupe CH=CH cis ou trans, dans lequel une unité CH peut être remplacée par un atome d'azote, un groupe C≡C acétylénique, un radical phényle divalent, un radical pyridine divalent, un radical thiophène divalent, un radical naphtalène divalent dans lequel un ou deux groupes CH peuvent être remplacés par des atomes d'azote, et un radical anthracène divalent dans lequel un ou deux groupes CH peuvent être remplacés par des atomes d'azote ;
où il peut également s'agir, pour une unité $CH_2$ qui peut être remplacée, d'une unité en position terminale dans un radical alkyle/une chaîne alkyle, c'est-à-dire d'une unité correspondante dans un groupe $-CH_3$ ;
et les groupes $CH_2$, sur lesquels, comme décrit ci-dessus, un atome d'hydrogène est remplacé, pouvant également être liés les uns aux autres en formant un cycle.

2. Nitrones de bisimide de l'acide pérylènetétracarboxylique de formule générale (II),

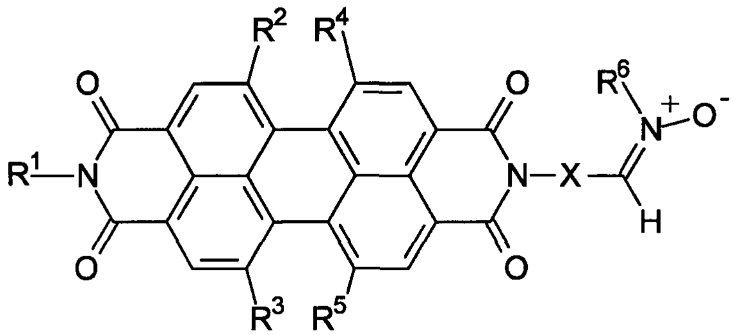

(II)

dans laquelle les radicaux $R^1$ à $R^6$ et X présentent la signification indiquée pour la formule (I).

3. Composé selon la revendication 1 ou 2, les radicaux $R^1$ à $R^5$ étant choisis parmi hydrogène et un radical hydrocarboné.

4. Composé selon l'une quelconque des revendications 1 à 3, $R^6$ étant choisi parmi un radical aryle ou un radical hétéroaryle.

5. Composé selon l'une quelconque des revendications 1 à 4, X étant un groupe qui est formé par un ou deux groupes phénylène ou un groupe bisphénylène en combinaison avec 1 à 4 unités méthylène.

6. Utilisation d'isoxazolidines de formule générale (IV) comme groupe donneur d'électrons dans des systèmes induits par la lumière pour la séparation de charges,

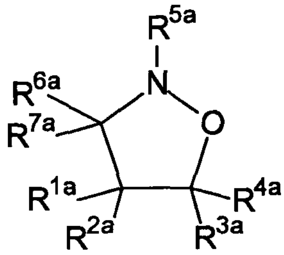

(IV)

les radicaux $R^{1a}$ à $R^{7a}$ étant identiques ou différents et représentant, indépendamment les uns des autres, hydrogène ou un radical alkyle linéaire comprenant au moins un et au plus 37 atomes de carbone ;
une à 10 unités $CH_2$ pouvant être remplacées indépendamment les unes des autres dans le radical alkyle par un groupe carbonyle, un atome d'oxygène, un atome de soufre, un atome de sélénium, un atome de tellure, un groupe CH=CH cis ou trans, dans lequel une unité CH peut également être remplacés par un atome d'azote, un groupe C≡C acétylénique, un radical phényle divalent, un radical pyridine divalent, un radical thiophène divalent, un radical naphtalène divalent dans lequel un ou deux groupes CH peuvent être remplacés par des atomes d'azote et un radical anthracène divalent, dans lequel un ou deux groupes CH peuvent être remplacés par des atomes d'azote ;
jusqu'à 12 atomes d'hydrogène individuels des groupes $CH_2$ pouvant être remplacés, à chaque fois indépendamment les uns des autres, même sur des atomes de carbone identiques, par les halogènes fluor, chlore, brome ou iode, un groupe cyano ou une chaîne alkyle linéaire comprenant jusqu'à 18 atomes de carbone, dans laquelle une à 6 unités $CH_2$ peuvent être remplacées indépendamment les unes des autres par un groupe carbonyle, un atome d'oxygène, un atome de soufre, un atome de sélénium, un atome de tellure, un groupe CH=CH cis ou trans, dans lequel une unité CH peut être remplacée par un atome d'azote, un groupe C≡C acétylénique, un radical phényle divalent, un radical pyridine divalent, un radical thiophène divalent, un radical naphtalène divalent dans lequel un ou deux groupes CH peuvent être remplacés par des atomes d'azote, et un radical anthracène divalent dans lequel un ou deux groupes CH peuvent être remplacés par des atomes d'azote ;
jusqu'à 12 atomes d'hydrogène individuels des groupes $CH_2$ dans un radical alkyle pouvant être remplacés, à chaque fois indépendamment les uns des autres, même sur des atomes de carbone identiques, par les halogènes fluor, chlore, brome ou iode, un groupe cyano ou une chaîne alkyle linéaire comprenant jusqu'à 18 atomes de carbone, dans laquelle une à 6 unités $CH_2$ peuvent être remplacées indépendamment les unes des autres par un groupe carbonyle, un atome d'oxygène, un atome de soufre, un atome de sélénium, un atome de tellure, un groupe CH=CH cis ou trans, dans lequel une unité CH peut être remplacée par un atome d'azote, un groupe C≡C acétylénique, un radical phényle divalent, un radical pyridine divalent, un radical thiophène divalent, un radical naphtalène divalent dans lequel un ou deux groupes CH peuvent être remplacés par des atomes d'azote, et un radical anthracène divalent dans lequel un ou deux groupes CH peuvent être remplacés par des atomes d'azote ;
les groupes $CH_2$, sur lesquels, comme décrit ci-dessus, un atome d'hydrogène est remplacé, pouvant également être liés les uns aux autres en formant un cycle ;
où il peut également s'agir, pour une unité $CH_2$ qui peut être remplacée, d'une unité en position terminale dans un radical alkyle/une chaîne alkyle, c'est-à-dire d'une unité correspondante dans un groupe $-CH_3$ ;
et les radicaux $R^{1a}$ à $R^{4a}$ et $R^{6a}$, à $R^{7a}$ pouvant en outre signifier, indépendamment les uns des autres, les atomes d'halogène F, Cl, Br ou I ou CN.

**7.** Procédé pour la préparation d'une dyade de pérylène-bisimide de formule (I) selon l'une quelconque des revendications 1 ou 3 à 5, une oléfine étant transformée par une cycloaddition 1,3-dipolaire avec une nitrone de formule (II) selon la revendication 2.

**8.** Procédé pour la préparation d'une nitrone de pérylène-bisimide de formule (II) selon l'une quelconque des revendications 2 à 5, un aldéhyde de pérylène-bisimide étant transformé avec un dérivé d'hydroxylamine.

**9.** Cellule photovoltaïque, comprenant un composé selon l'une quelconque des revendications 1 à 5.

**10.** Cellule photovoltaïque selon la revendication 9, où il s'agit d'une cellule à base de dioxyde de titane.

**11.** Utilisation d'un composé selon l'une quelconque des revendications 1 à 5 comme système pour la séparation des charges induite par la lumière lors de la production d'énergie solaire.

**12.** Utilisation d'un composé selon l'une quelconque des revendications 1 à 5 comme agent de réduction ou d'oxydation induit par la lumière dans des réactions chimiques.

**13.** Utilisation d'un composé selon l'une quelconque des revendications 1 à 5 comme indicateur de fluorescence pour des acides protoniques ou des acides de Lewis.

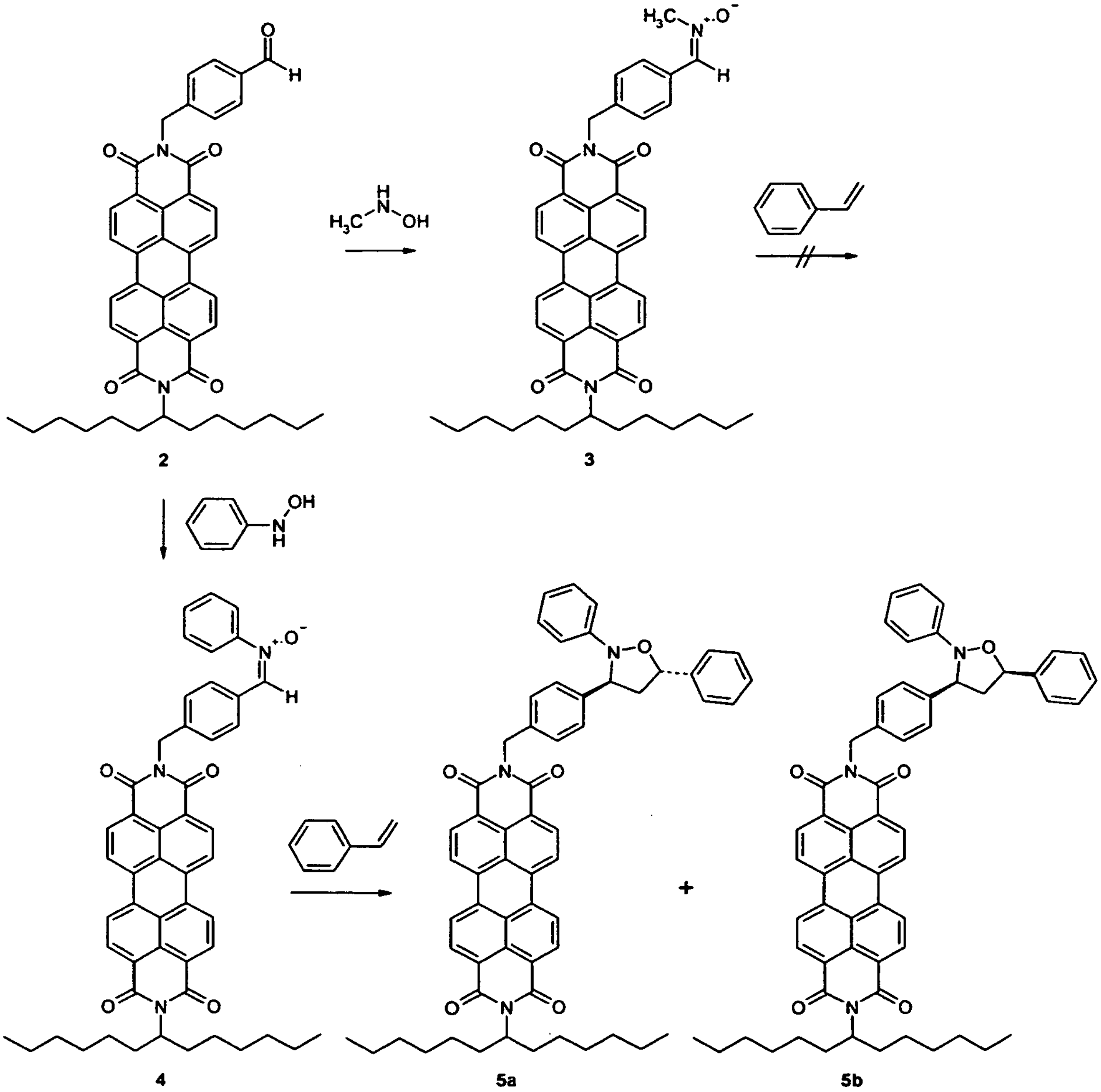

2

3

4

5a

+

5b

Figur 1.

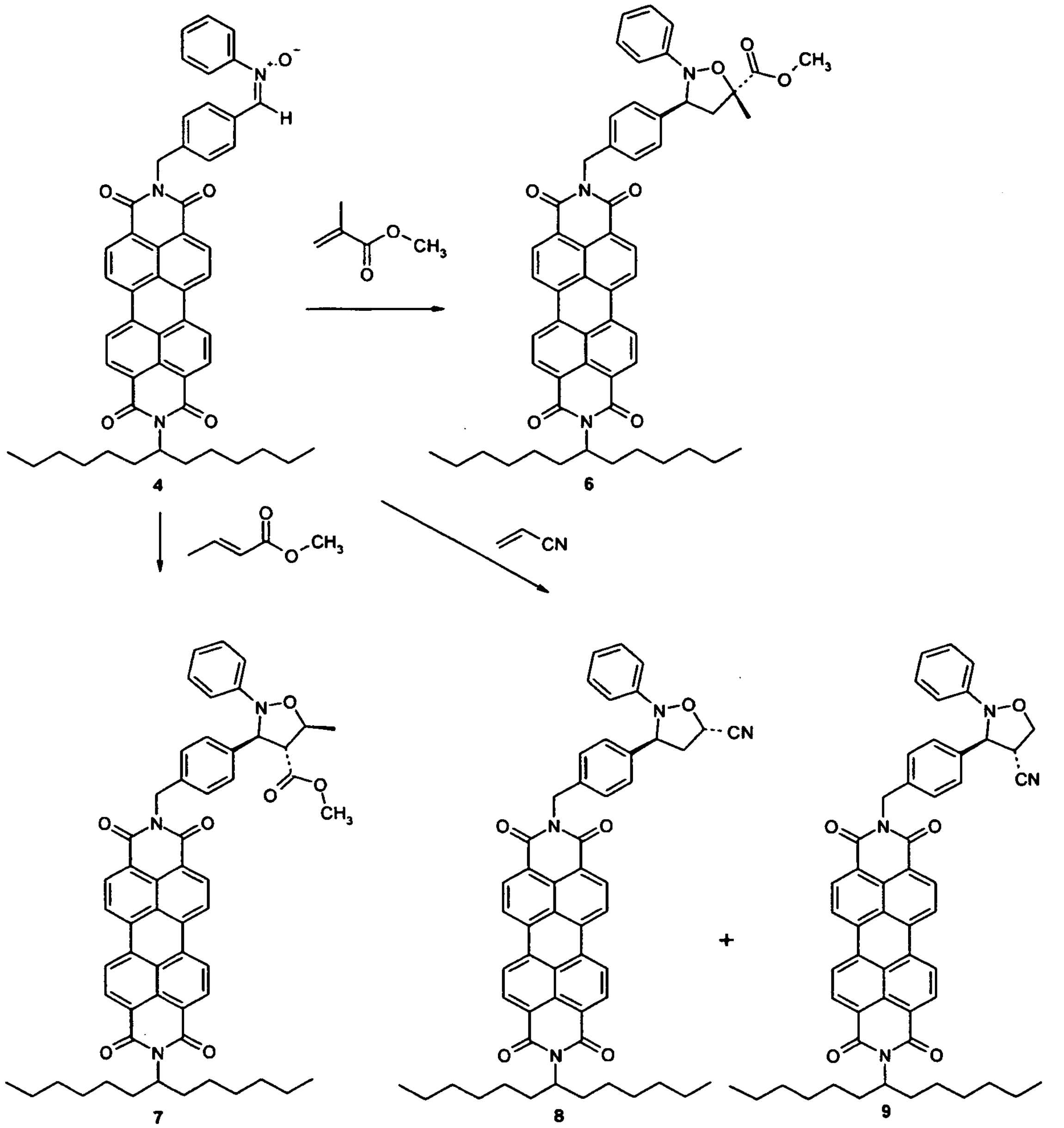

4

6

7

8

9

Figur 2.

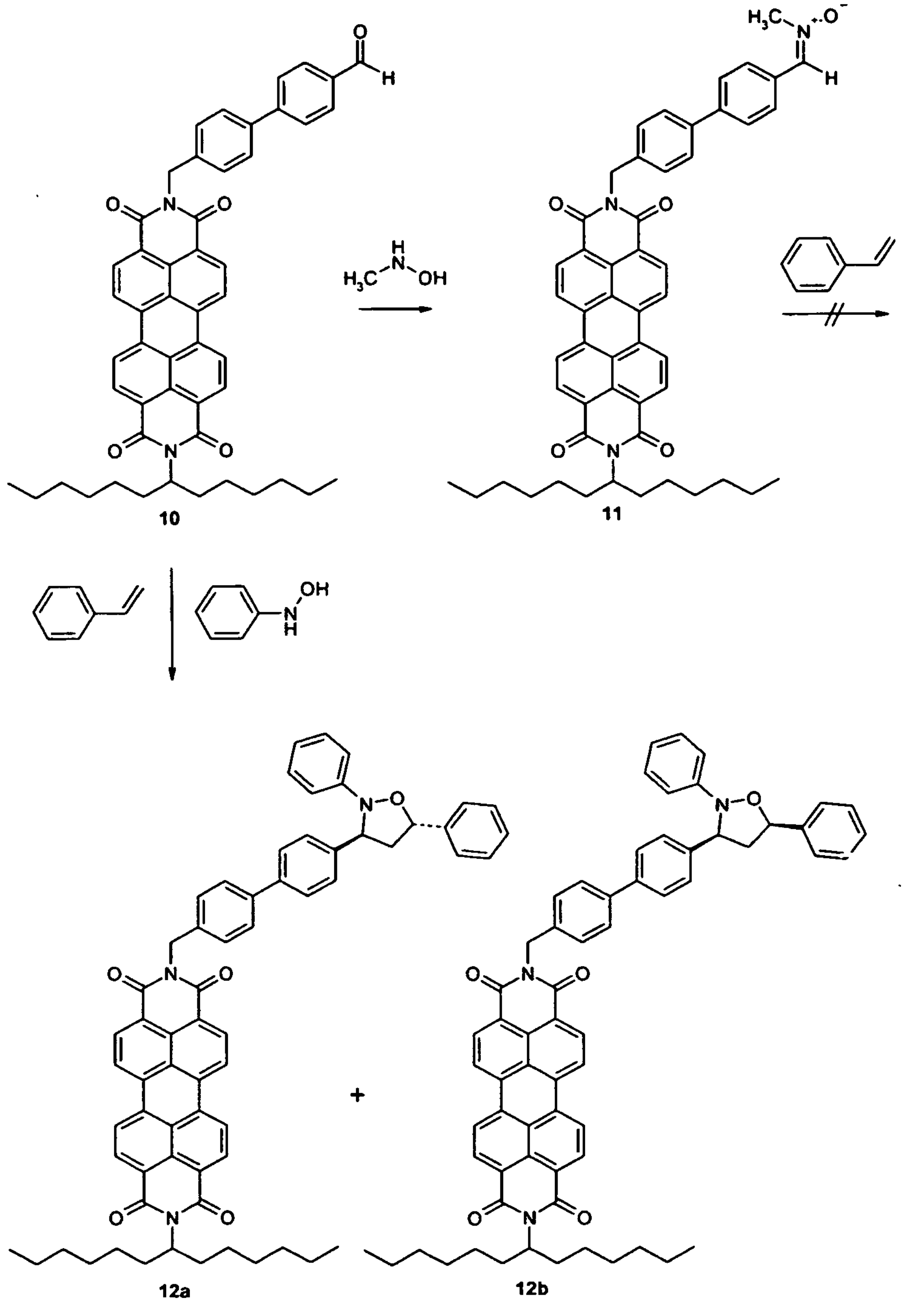

10

11

12a

+

12b

Figur 3.

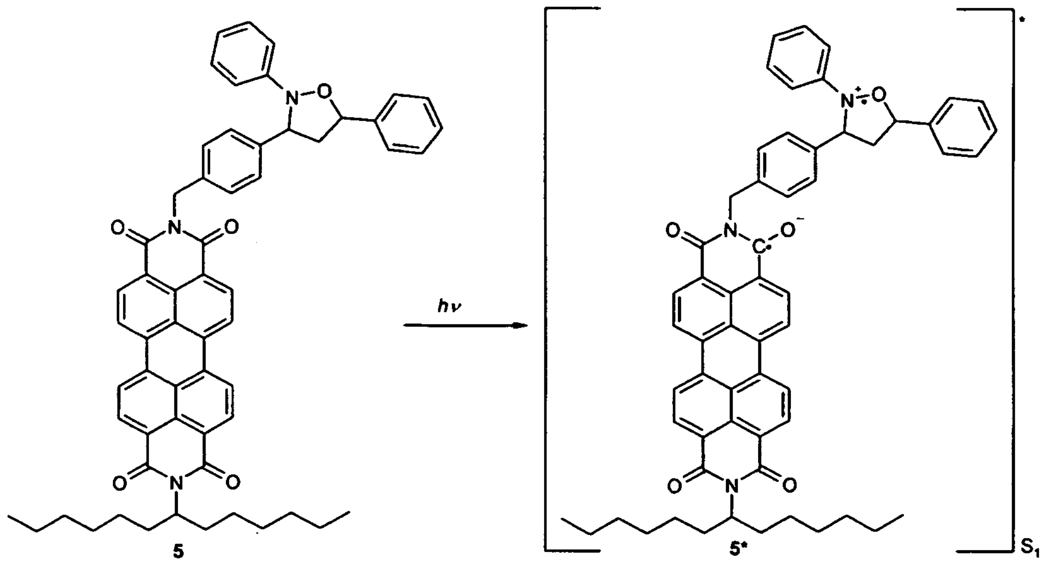


5 5*

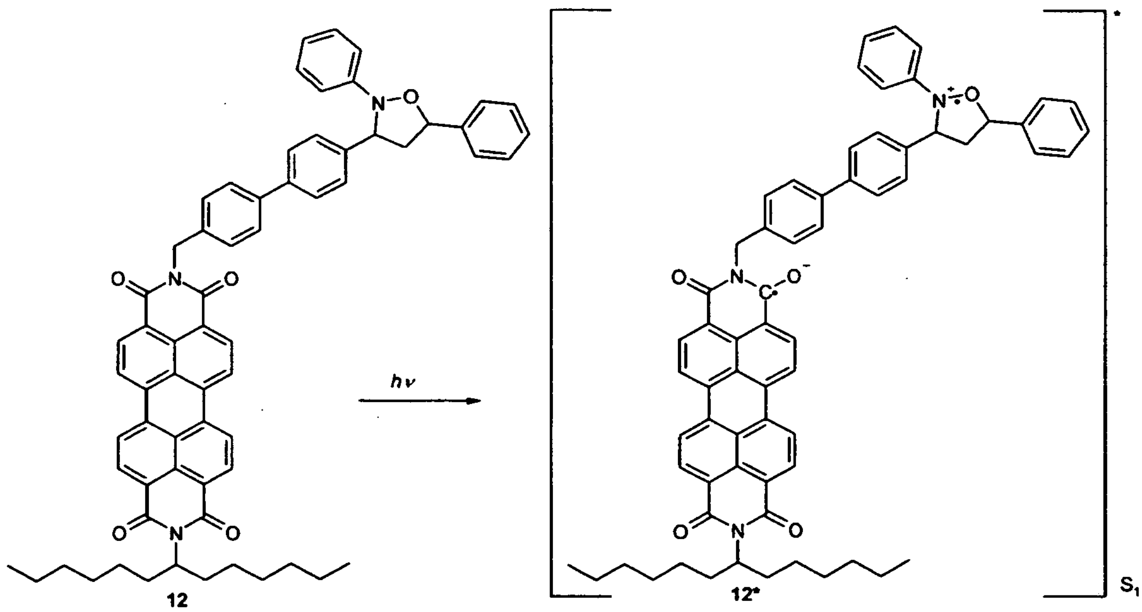


12 12*

Figur 4.

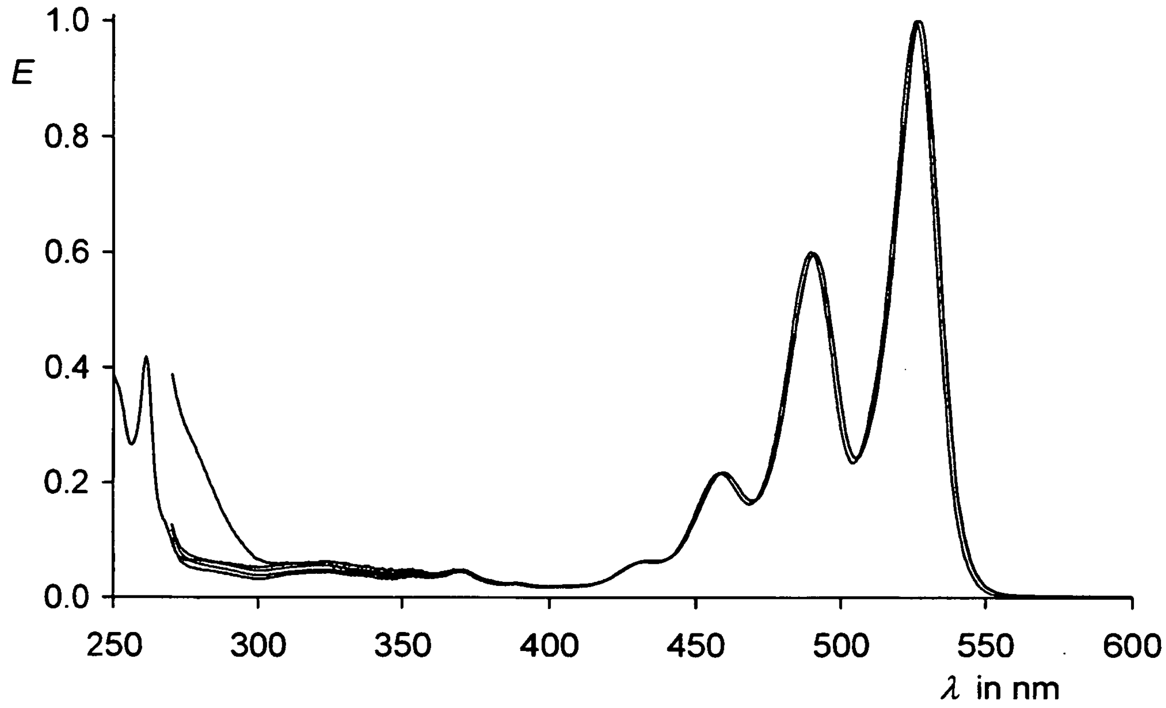


Figur 5

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- JP 8065635 A


### In der Beschreibung aufgeführte Nicht-Patentliteratur


- **L. FLAMIGNI ; B. VENTURA ; M. TASIOR ; T. BECHERER ; H. LANGHALS ; D. T. GRYKO.** *Chem. Eur. J.,* 2008, vol. 14, 169-183
- **M. TASIOR ; D. T. GRYKO ; JING SHEN ; K. M. KADISH ; T. BECHERER ; H. LANGHALS ; B. VENTURA ; L. FLAMIGNI.** *J. Phys. Chem.,* 2008, vol. 112, 19699-19709
- **H. LANGHALS ; W. JONA.** *Chem. Eur.J.,* 1998, vol. 4, 2110-2116
- **H. LANGHALS ; A. OBERMEIER.** *Eur. J. Org. Chem.,* 2008, vol. 36, 6144-6151
- **H. LANGHALS.** *Helv. Chim. Acta,* 2005, vol. 88, 1309-1343
- **H. LANGHALS.** *Heterocycles,* 1995, vol. 40, 477-500
- **H. LANGHALS.** Molecular Devices. Chiral, Bichromophoric Silicones: Ordering Principles in Complex Molecules. Springer, 2008, 51-63
- **H. LANGHALS ; J. KAROLIN ; L. B.-Ä. JOHANSSON.** *J. Chem. Soc., Faraday Trans.,* 1998, vol. 94, 2919-2922
- **S. DEMMIG ; H. LANGHALS.** *Chem. Ber.,* 1988, vol. 121, 225-230
- **H. LANGHALS ; S. DEMMIG ; T. POTRAWA.** *J. Prakt. Chem.,* 1991, vol. 333, 733-748
- **P. GRÜNANGER ; P. VITA-FINZI.** Isoxazolidines, in The Chemistry of Heterocyclic compounds. Wiley, 1991, vol. 49/1, 649-877
- **S. CICCHI ; F. M. CORDERO ; D. GIOMI.** *Five-membered ring systems: with O & N atoms in Progress in Heterocyclic Chemistry,* 2003, vol. 15, 261-283
- **Y. TAKEUCHI ; F. FURUSAKI.** The chemistry of isoxazolidines. *Advances in Heterocyclic Chemistry,* 1977, vol. 21, 207-251
- **H. LANGHALS ; T. BECHERER ; J. LINDNER ; A. OBERMEIER.** *Eur. J. Org. Chem.,* 2007, vol. 26, 4328-4336
- **K. RUECK-BRAUN ; T. E. H. FREYSOLDT ; F. WIERSCHEM.** *Chem. Soc. Revs.,* 2005, vol. 34, 507-516
- **H. LANGHALS ; T. BECHERER ; J. LINDNER ; A. OBERMEIER.** *Eur. J. Org. Chem.,* 2007, 4328-4336